Europäisches Patentamt

**European Patent Office**

**Office européen des brevets**

(19)

(11) Publication number: **0 171 908**
**A2**

(12) # EUROPEAN PATENT APPLICATION

(21) Application number: **85304735.5**

(22) Date of filing: **03.07.85**

(51) Int. Cl.⁴: **C 12 N 15/00**
**C 12 N 1/18, C 12 N 1/20**
**C 07 K 13/00, C 12 P 21/02**

(30) Priority: **11.07.84 PC T/JP84/00356**
**04.09.84 PC T/JP84/00423**
**12.12.84 PC T/JP84/00585**
**03.06.85 PC T/JP85/00306**

(43) Date of publication of application:
**19.02.86 Bulletin 86/8**

(84) Designated Contracting States:
**AT BE CH DE FR GB IT LI LU NL SE**

(71) Applicant: **Takeda Chemical Industries, Ltd.**
**27, Doshomachi 2-chome Higashi-ku**
**Osaka-shi Osaka, 541(JP)**

(72) Inventor: **Kikuchi, Masakazu**
**4-16, Higashitokiwadai 7-chome Toyono-cho**
**Toyono-gun Osaka 563-01(JP)**

(72) Inventor: **Fujisawa, Yukio**
**31-104,1 Mikagenakamachi 4-chome Higashinada-ku**
**Kobe-shi Hyogo 658(JP)**

(72) Inventor: **Ikeyama, Shuichi**
**62, Kasaboko-cho Shijodori Nishitouin-nishi-iru**
**Shimogyo-ku Kyoto-shi Kyoto 600(JP)**

(72) Inventor: **Nishimura, Osamu**
**123-502, 2 Higashitoyonakacho 5-chome**
**Toyonaka-shi Osaka 560(JP)**

(74) Representative: **Lewin, John Harvey et al,**
**Elkington and Fife High Holborn House 52/54 High**
**Holborn**
**London WC1V 6SH(GB)**

(54) Hepatitis B virus surface antigen and production thereof.

(57) Hepatitis B virus surface antigen P31 can be produced by cultivating a transformant carrying a recombinant DNA wherein a DNA coding for hepatitis B virus surface antigen P31 is inserted into the 3' end of a promoter region.

EP 0 171 908 A2

0171908

HEPATITIS B VIRUS SURFACE ANTIGEN AND PRODUCTION THEREOF

Technical Field

This invention relates to hepatitis B virus surface antigen and production thereof. More particularly, this invention relates to (1) a recombinant DNA wherein a DNA coding for hepatitis B virus surface antigen P31 is inserted into the 3' end of a promoter, (2) production of said recombinant DNA, (3) a transformant carrying said recombinant DNA, (4) production of said transformant, (5) non-glycosylated hepatitis B virus surface antigen P31 protein and (6) production of hepatitis B virus surface antigen P31.

Background Art

Type B hepatitis is a viral disease frequently encountered in particular in tropical Africa, Southeast Asia and Far East and, epidemiologically, it is suggested that hepatitis B may cause chronic hepatitis, hepatic cirrhosis and, further, primary liver cancer. The pathogen is hepatitis B virus (hereinafter referred to as HBV for short), one of DNA viruses, which occurs as spherical particles having a diameter of 42 nm as called Dane particles after the discoverer. In the external layer of said particles, there exists HBV surface antigen (hereinafter referred to as HBsAg for short), which is classifiable into subtypes such as adr, adw, ayr and ayw according to the differing antigenicity, and the subtypes adw and adr are popular in Japan.

In the blood of patients with hepatitis B, tiny particles and tubular particles are detected in addition to Dane particles and, in these particles, there is observed the same type of HBsAg as found in Dane particles. It is known, with other viruses, that an antibody to the surface antigen of a virus can prevent the infection with said virus and, in the case of HBV, too, there seems to be the possibility of vaccine production against hepatitis B on the basis of HBsAg. However, human and chimpanzee are the only animal species that can be infected with HBV, so that all the attempts so far made to cause infection of cultured cells with HBV have failed. Therefore, for the time being, the blood of human infected patients is the only available source of HBsAg and the small-sized particles obtained can satisfy only the requirement for its use as a material for diagnostic reagents but are by no means sufficient for large-scale vaccine production.

Recent advancements in molecular biology have already made it possible to transform bacteria by introducing a DNA coding for a nonbacterial protein thereinto. If the expression of the structural gene coding for HBsAg (hereinafter referred to as HBsAg gene for short) in bacteria becomes possible as a result of application of recombinant DNA techniques, large-quantity production of HBsAg free of the risk of HBV infection will expectedly become possible and there will be opened the way for practical use of the hepatitis B vaccine.

As for the subtype ayw, which is most frequently encountered in Europe and America among the currently known four subtypes adw, adr, ayw and ayr, the location and base sequence of the HBsAg gene have been determined [Galibert, F. et al., Nature, 281, 646 (1979); Charnay, P. et al., Nucleic Acids Res., 7, 335 (1979)] and the expression of said gene as a hybrid protein in Escherichia coli has been reported [Charnay, P. et al., Nature, 286, 893 (1980); Edman, J. C. et al., Nature, 291, 503 (1981)].

As regards the subtypes adw and adr, which are encountered frequently in Japan, some of the present inventors successfully created a DNA containing the HBsAg gene, determined the DNA base sequence of said gene and the location thereof on the genome and, furthermore, opened the way for large-quantity production of HBsAg by cultivating a transformant carrying said recombinant DNA (Japanese published unexamined patent applications Nos. 194897/1983, 201796/1983 and 74985/1984).

Recently, Machida, A. et al. [Gastroenterology, 85, 268 (1983); ibid., 86, 910 (1984)] demonstrated that, in the small-size particles of HBsAg obtained from the plasma of patients positively responding to the hepatitis B virus e antigen, there exist the P31 protein (molecular weight 31 kilodaltons) and P35 protein (conjugated protein which is sugar-bound P31 and has a molecular weight of 35 kilodaltons) in addition to the so-far identified main peptides such as P-I (molecular weight 22-24 kilodaltons) and P-II (molecular weight 25-29.5 kilodaltons) [Peterson, D. L. et al., Proc. Natl. Acad. Sci. USA, 74, 1530 (1977)]. P31 is composed of P-I and 55 amino acid residues of the pre-S region as added to the N-terminal of P-I, and it has also been demonstrated that the polymerized human serum albumin (poly-HSA) receptor occurs in said region. Furthermore, the 1984 report cited above shows that the above-mentioned P31 protein has a sugar chain. On the other hand, it is considered that, since said receptor is also found on the hepatocyte surface, the proliferation takes place after the occurrence of Dane particle-hepatocyte adhesion through poly-HSA. Therefore, it is expected that the HBV infection can be prevented more efficiently if the poly-HSA receptor on Dane particles can be masked with an antibody to P31, since, said particles are no more able to bind with hepatocytes.

Disclosure of the Invention

This invention relates to hepatitis B virus surface antigen and production thereof. More particularly, this invention provides (1) a recombinant DNA wherein a DNA coding for hepatitis B virus surface antigen P31 is inserted into the 3' end of a promoter, (2) production of said recombinant DNA, (3) a transformant carrying said recombinant DNA, (4) production of said transformant, (5) non-glycosylated hepatitis B virus surface antigen P31 protein and (6) production of hepatitis B virus surface antigen P31.

Further, this invention provides a method for producing the hepatitis B virus surface antigen P31 protein which comprises cultivating a transformant carrying a DNA having a base sequence coding for hepatitis B virus surface antigen P31, accumulating the hepatitis B virus surface antigen P31 in the culture, collecting the P31-containing solution and subjecting the P31-containing solution to a purification process involving affinity-chromatographic treatment.

- 5 -                               0171908

The DNA coding for the hepatitis B virus surface antigen P31, which is to be used in the practice of the invention, may be of any subtype (adr, adw, ayr or ayw) and can be prepared, for example, by the methods described hereinbelow. Thus, the plasmid pBR322-EcoRI/HBV933 (hereinafter referred to as pHBV933 for short) with the 3.2 kb subtype adw HBV DNA inserted therein, which is described in Japanese published unexamined patent application (Kokai) No. 194897/1983 or in Nucleic Acids Res., 11, 1747 (1983), is subjected to double digestion with the restriction enzymes KpaI and EcoRI, to give a 961 bp DNA fragment which contains a part of the pre-S region. A DNA coding for P31 can be constructed by joining, to said fragment, an appropriate adapter containing the sequence [ $_{3'}^{5'}$ $\frac{ATGCAGTGG}{TACGTCACCTTAA}$ $_{5'}^{3'}$ ]. Alternatively, the plasmid pBR322-BamHI/HBr330 (hereinafter referred to as pHBr330 for short) with the 3.19 kb subtype adr HBV DNA inserted therein, which is described in Japanese published unexamined patent application No. 74985/1984 or in Nucleic Acids Res., 11, 1747 (1983), is subjected to double digestion with the restriction enzymes EcoRI and BamHI, whereby a 1398 bp DNA fragment containing a part of the pre-S region can be obtained. A DNA coding for P31 can be prepared by joining the above-mentioned adapter to this fragment.

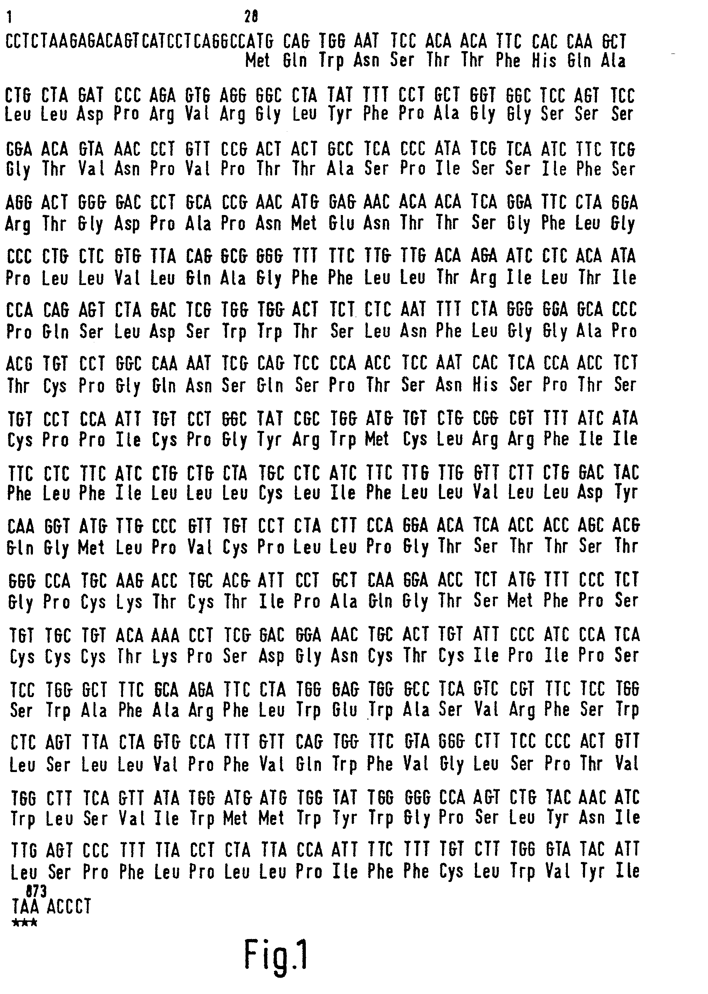

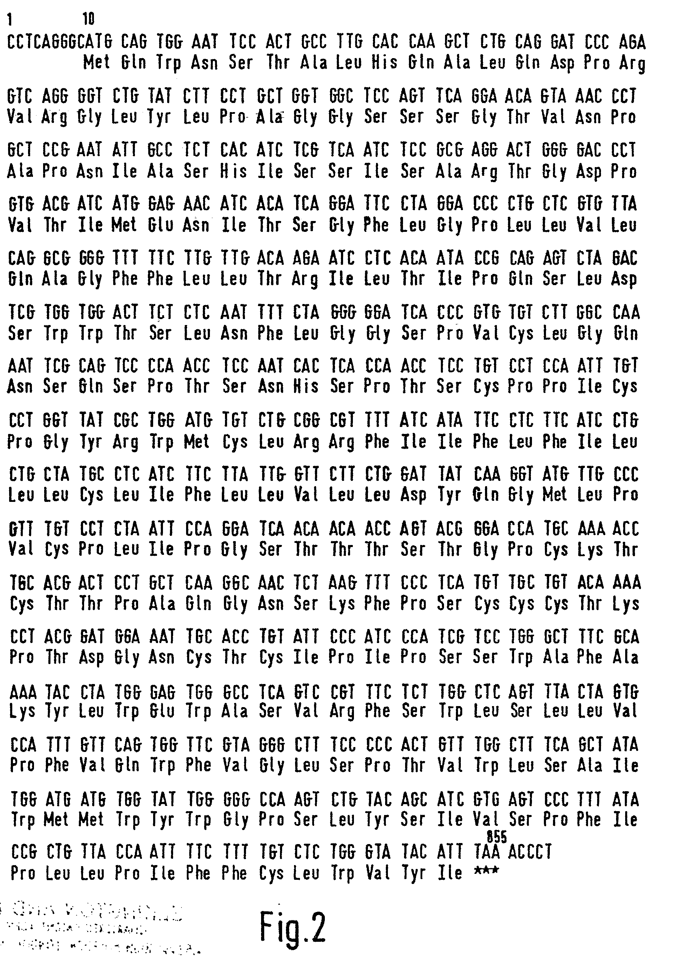

The DNA coding for subtype adw HBsAg P31 is, for example, a DNA comprising the base sequence from the base pair No. 28 to No. 873 of the DNA sequence as shown in Fig. 1, and the DNA coding for subtype adr HBsAg P31 is, for example, a DNA comprising the base sequence from the base pair No. 10 to No. 855 of the DNA sequence as shown in Fig. 2.

The P31-encoding DNA may be either of the viral origin or a chemically synthesized one.

The DNA coding for subtype ayr or ayw HBsAg P31 can be prepared according to the same method as mentioned above.

A recombinant DNA which allows the expression of the P31-encoding DNA can be constructed by insertion of this P31-encoding DNA at the 3'-end of a promoter region capable of functioning in each host (e.g. Escherichia coli, Bacillus subtilis, yeast, animal cells).

The promoter region may be any region which contains a site necessary for RNA polymerase binding and for the initiation of mRNA synthesis.

For use of a strain of Escherichia coli, for instance, as the host, a recombinant DNA which allows the expression of the P31-encoding DNA can be constructed by insertion of the P31-encoding DNA at the 3'-end of a promoter region capable of functioning in Escherichia coli. For instance, the P31-encoding DNA is inserted into an expression vector, such as pTRP601 or pTRP771, which is mentioned in Japanese published unexamined patent application No. 201796/1983, with the aid of T4 DNA ligase. A strain of Escherichia coli (e.g. the strain C600, 294, W3110, RR1 or PR13) is transformed with the mixture by the known method [Cohen, S. N. et al., Proc. Natl. Acad. Sci. USA, 69, 2110 (1972)] or a modification thereof.

The promoter to be used is not necessarily limited to the trp promoter (trp-p) but, for instance, the recA promoter [Japanese published unexamined patent application No. 65099/1984], lac promoter and $\lambda P_L$ promoter may also be used.

The transformant carrying the novel recombinant DNA containing the P31-encoding DNA as obtained in the above manner can be selected by using, for example, such phenotype as ampicillin resistance, tetracycline resistance or both ampicillin and tetracycline resistance. For finding out the strain carrying the novel recombinant plasmid DNA which contains the P31-encoding DNA from among the drug-resistant transformants obtained, the following technique, for instance, is used: One chain of the above-mentioned adapter $^{5'}$AATTCCACTGCATTGTAT$^{3'}$ is labelled radioisotopically using $\gamma$-$^{32}$P-ATP and T4 polynucleotide kinase. By using this as the probe and using the per se known colony hybridization method [Grunstein, M. and Hogness, D. S., Proc. Natl. Acad. Sci. USA, 72, 3961 (1975)], a positively responding clone can surely be found out from among the drug-resistant transformants already obtained.

The thus-selected transformant is cultivated in a per se known medium. As the medium, there may be mentioned, for example, L broth, Penassay broth, and M-9 medium supplemented with glucose and casamino acids [Miller, J., Experiments in Molecular Genetics, 431-433 (Cold Spring Harbor Laboratory, New York, 1972)]. There can be added, as necessary, such an agent as 3β-indolylacrylic acid for efficient promoter functioning.

The cultivation of said transformant is carried out generally at 15°C to 43°C, preferably at 28°C to 40°C, for 2 to 24 hours, preferably for 4 to 16 hours, if necessary with aeration and/or stirring.

When a yeast, for instance, is used as the host, the yeast transformant can be prepared in the following manner. Into the Escherichia coli-yeast shuttle vector YEp13 [Broach, J. R. et al., Gene, 8, 121 (1979)], pSH15 or pSH19 [Harashima, S. et al., Mol. Cell. Biol., 4, 771 (1984)], there is inserted a yeast promoter region such as the repressible acid phosphatase gene promoter region [Meyhack, B. et al., EMBO J., 6, 675 (1982)], the promoter region of the glyceraldehyde-3-phosphate dehydrogenase gene [Holland, J. P. and Holland, M. J., J. Biol. Chem., 255, 2596 (1980)] or the promoter region of the 3-phosphoglycerate kinase gene [Dobson, M. J. et al., Nucleic Acids Res., 10, 2625 (1982)], and the P31-encoding DNA is joined thereto just behind said region under the action of T4 DNA ligase. Using this reaction mixture, the above-mentioned host strain of Escherichia coli is transformed by the method of Cohen et al. cited above. The thus-produced transformant which carries the novel recombinant DNA containing the P31-encoding DNA can be selected by using ampicillin resistance as the phenotype. For finding out the strain carrying the novel recombinant plasmid DNA having the P31-encoding DNA from among the resistant strains obtained, the above-mentioned method is used in the same manner.

The plasmid DNA is isolated from the thus-selected transformant by the alkaline extraction method [Birnboim, H. C. and Doly, J., Nucleic Acids Res., $\underline{7}$, 1513 (1979)] and used for the transformation of a yeast, for example a leucine-requiring strain of Saccharomyces cerevisiae such as AH22R$^-$ (leu2 his4 can1 cir$^+$ pho80) [Proc. Natl. Acad. Sci. USA, $\underline{80}$, 1 (1983)] or AH22R$^-$-derived K33-7B (pho80-AH22, pho8-2) or K33-8D (pho80-AH22, pho8-2 trp1) by the known method [Hinnen, A. et al., Proc. Natl. Acad. Sci. USA, $\underline{75}$, 1927 (1978)] or a modification thereof. The yeast as the host is not limited to these but preferably is a strain of Saccharomyces cerevisiae.

The yeast transformant obtained is cultivated on a per se known medium. As the medium, there may be mentioned, for example, Burkholder minimum medium [Bostian, K. L. et al., Proc. Natl. Acad. Sci. USA, $\underline{77}$, 4505 (1980)].

The cultivation of the yeast transformant obtained is carried out generally at 15°C to 40°C, preferably at 24°C to 37°C, for 10 to 96 hours, preferably for 24 to 72 hours, if necessary with aeration and/or stirring.

For using Bacillus subtilis or animal cells, for instance, as the host, the P31-encoding DNA is inserted at the 3'-end of a promoter region capable of functioning in Bacillus subtilis or animal cells, and the host is transformed with the resultant recombinant DNA. P31 can be produced by cultivating the transformant. However, Escherichia coli and a yeast are more preferable as the host.

The product P31 may be either in the glycosylated form or in the unglycosylated form. P31 obtainable from the transformants of Escherichia coli is substantially unglycosylated, but one molecule of carbohydrate may be bound thereto per molecule of P31.

It is generally known that the growth of an HBsAg DNA-carrying transformant is inhbited by the production of surface antigen gene products. In accordance with the invention, however, the growth inhibition is removed and the yield of P31 is increased as a result of the use of the P31-encoding DNA.

The P31 activity of the product can be determined, for example, by the direct immunoassay method [Fujisawa, Y. et al., Nucleic Acids Res., 11, 3581 (1983)] which comprises binding the sample to a cyanogen bromide-activated cellulose paper followed by reaction with $^{125}$I-anti-HBsAg antibody of Ausria II-125 (Dainabbott).

After cultivation, cells are collected by the conventional method. In the case of a transformant of Escherichia coli, the cells may be subjected to an appropriate treatment such that the cells are suspended in a buffer containing a protein denaturing agent such as urea or guanidine hydrochloride and that the suspension is stirred at a cool place and then centrifuged to give a P31-containing supernatant, or that the cells are suspended in a buffer and disrupted by sonication, lysozyme, and/or freezing and thawing and then that a P31-containing supernatant is separated by centrifugation. It is preferable among others, however, to suspend the cells collected in a buffer, add lysozyme, homogenize for causing lysis, add a buffer containing urea (3-10 M), stir the mixture (at 0 to 10°C for 0.5 to 8 hours) and then collect a supernatant by centrifugation.

In the case of a yeast transformant, the cells are disrupted by using Zymolyase (Kirin Brewery Co.) or mechanically by using glass beads. Thereto is added a surfactant such as Triton X-100 or deoxycholate, or a protein denaturing agent such as guanidine hydrochloride, whereby P31 can be extracted more advantageously.

The separation and purification of the P31 protein from the above-mentioned extract is conducted in a purification process involving affinity-chromatographic treatment.

As the affinity chromatography, there may be mentioned affinity chromatography using polymerized human serum albumin (poly-HSA) as the ligand or antibody column treatment using an antibody to HBsAg, in particular a monoclonal antibody to HBsAg.

The affinity chromatography using poly-HSA as the ligand is used most advantageously for the purification of the P31 protein.

As the carrier in affinity chromatography, there may be used, for instance, Formyl-Cellulofine (Seikagaku Kogyo) or Affi-Gel 15 (Bio-Rad), and Formyl-Cellulofine is preferable among others.

Poly-HSA can be produced by polymerizing human serum albumin using a crosslinking agent (e.g. glutaraldehyde). This is allowed to bind to the above carrier using a reducing agent (e.g. $NaCNBH_3$), for instance, and the carrier-poly-HSA coupling product obtained, after washing if desired, is packed generally into a column for its use.

For purifying the P31 protein by affinity chromatography with poly-HSA as the ligand, the above-mentioned P31-containing solution (cell extract supernatant) is allowed to be adsorbed on the above-mentioned column equilibrated beforehand with a buffer [e.g. phosphate buffer] and then eluted with a buffer. Said buffer may contain an appropriate amount of a surfactant (e.g. Tween 20) or a protein denaturing agent (urea), for instance, and can be used as an adequate eluent by modifying the combination of such additives and the concentrations thereof.

The P31 protein-containing eluate fractions are collected and, as desired, concentrated by ultrafiltration, for instance.

It is preferable that the above concentrate, desirably after reduction with an SH reagent such as dithiothreitol, be further subjected to chromatographic treatment using a hydrophobic column, such as high-performance liquid chromatography using a reversed-phase column or hydrophobic chromatography.

As the carrier in the above-mentioned high-performance liquid chromatography, there may be mentioned alkylated ($C_1$ to about $C_{18}$) silicone type carriers, for example AP-202 300 $\overset{o}{A}$ ($C_8$) and AP-224 300 $\overset{o}{A}$ ($C_8$) (YMC-Shimakyu), Ultrapore RPSC (Beckman) and Hi-Pore RP-34 (Bio-Rad). Among others AP-202 300 $\overset{o}{A}$ ($C_8$) and AP-224 300 $\overset{o}{A}$ ($C_8$) are preferable and AP-224 300 $\overset{o}{A}$ ($C_8$) is more preferable.

As the carrier in hydrophobic chromatography, there may by mentioned alkylated ($C_1$ to about $C_{18}$) carriers, for exmaple Butyl-Toyopearl 650M (Toyo Soda Manufacturing) and Octyl-Sepharose CL-4B (Pharmacia). The use of high-performance liquid chromatography using a reversed-phase column is particularly advantageous.

In the above-mentioned high-performance liquid chromatography using a reversed-phase column, water, a $C_1$ to $C_6$ lower alkanol (e.g. ethanol, propanol), acetonitrile or the like is preferably used as the eluent, with the pH adjusted to 1.2 to 5.0 with trifluoroacetic acid, for instance. The rate of elution is preferably 0.1 to 100 ml/min, more preferably 0.5 to 30 ml/min.

The P31 protein-containing fraction thus obtained may be lyophilized as desired to give a white powder.

For determining the purity (specific acitivity) of the thus-produced P31 protein, there can be used, for example, the enzyme immunoassay (ELISA) method which comprises reacting the sample with a poly-HSA-coated plastic plate and detecting the poly-HSA-bound P31 protein using a horseradish peroxidase (HRP)-coupled anti-HBsAg monoclonal antibody, or the radioimmunoassay (RIA) method which comprises detecting the poly-HSA-bound P31 protein using $^{125}$I-anti-HBsAg antibody of Ausria II-125 (Dainabbott, USA).

The use of the production method according to the invention can give highly purified
and substantially pure P31 protein suitable for use as a drug, for instance.

For example, by purifying P31 produced in <u>Escherichia</u> <u>coli</u> carrying a DNA having a base sequence coding for HBsAg P31 using the method of the invention, there can be obtained the HBsAg P31 protein which is substantially pure and has the following characteristic properties:

(1)   In SDS-polyacrylamide slab gel electrophoresis, it is homogeneous and its molecular weight as determined by said electrophoresis (under reducing conditions) is 32,000 $\pm$ 1,000 daltons.

(2)   It contains methionine (or formylated methionine) or glutamine as the amino-terminal amino acid.

(3)   It contains isoleucine as the carboxy-terminal amino acid.

(4)   It binds poly-HSA.

(5)   It has a specific acitivity of not less than 1 x 10$^6$ units/mg.

The substantially pure P31 protein produced by the method of the invention has the same biological activities as the known HBsAg small particles produced from the blood of HBV-infected patients as the raw material have, and can be used as a vaccine for the diagnosis, prevention and/or treatment of HBV infection in the same manner as said HBsAg small particles.

The determination of the activity (specific activity) of the P31 protein for the protein purity determination as described herein was performed by the ELISA method. Thus, a test solution (P31 protein-containing solution) was distributed, in 100-µl portions, into the wells of an Immunoplate II (Nunc) with the poly-HSA obtained in Reference Example 4-(1) physically adsorbed thereon in advance. After overnight reaction at 4°C, the plate was washed with PBS containing 5% bovine serum and 0.05% Tween 20 and, then, 100 µl of a horseradish peroxidase-bound anti-HBsAg monoclonal antibody solution was added to each well of the plate. After 2 hours of reaction at 37°C, the plate was washed again with the buffer mentioned above, followed by addition of 100 µl of citrate-phosphate buffer (pH 5.0) containing o-phenylenediamine (4 mg/10 ml) and aqueous hydrogen peroxide solution (4 µl/10 ml). After 30 minutes of reaction at room temperature, the enzymatic reaction was terminated by adding 50 µl of 2 N sulfuric acid and the absorbance at 492 nm was measured using a Titertek Multiskan MC. In this assay, the purified sample obtained in Example 7 was used as the standard and the results were given in terms of the number of units regarding the absorbance value (0.05 O.D.) given by 1 ng of the standard as representing 1 unit.

In the present specification, drawings and claims, the bases, amino acids and so on, when indicated by abbreviations, are indicated by the abbreviations according to the IUPAC-IUB Commission on Biochemical Nomenclature or by the conventional abbreviations in the relevant field. The following is a list of examples of such abbreviations. Where there is the possibility of optical isomerism relative to an amino acid, it is to be noted that said amino acid is in the L form unless otherwise specifically indicated.

| | |
|---|---|
| DNA | Deoxyribonucleic acid |
| RNA | Ribonucleic acid |
| mRNA | Messenger-RNA |
| A | Adenine |
| T | Thymine |
| G | Guanine |
| C | Cytosine |
| dATP | Deoxyadenosine triphosphate |
| dTTP | Deoxythymidine triphosphate |
| dGTP | Deoxyguanosine triphosphate |
| dCTP | Deoxycytidine triphosphate |
| ATP | Adenosine triphosphate |
| EDTA | Ethylenediaminetetraacetic acid |
| SDS | Sodium dodecylsulfate |
| DTT | Dithiothreitol |
| Gly | Glycine |

| | |
|---|---|
| Ala | Alanine |
| Val | Valine |
| Leu | Leucine |
| Ile | Isoleucine |
| Ser | Serine |
| Thr | Threonine |
| Cys | Cysteine |
| ½ Cys | Half cystine |
| Met | Methionine |
| Glu | Glutamic acid |
| Asp | Aspartic acid |
| Lys | Lysine |
| Arg | Arginine |
| His | Histidine |
| Phe | Phenylalanine |
| Tyr | Tyrosine |
| Trp | Tryptophan |
| Pro | Proline |
| Asn | Asparagine |
| Gln | Glutamine |
| $Ap^r$ | Ampicillin resistance gene |
| $Tc^r$ | Tetracycline resistance gene |
| $P_R$ | $\lambda P_R$ promoter |
| ars 1 | Autonomous replication sequence 1 |
| IR | Inverted repeat |

Brief Description of the Drawings

Fig. 1 shows a DNA sequence coding for subtype adr HBsAg P31 (upper row) and the corresponding amino acid sequence (lower row);

Fig. 2 shows a DNA sequence coding for subtype adw HBsAg P31 (upper row) and the corresponding amino acid sequence (lower row);

Fig. 3 is a construction scheme for the plasmid pPHO17-1, the symbols E, S, B, H and X standing for EcoRI, SalI, BamHI, HindIII and XhoI, respectively;

Fig. 4 is a construction scheme for the plasmid pPKT700-1, the symbols E, S, B, H and X standing for EcoRI, SalI, BamHI, HindIII and XhoI, respectively;

Fig. 5 is a construction scheme for the plasmid pGLD906-1, the symbols E, S, B, H and X standing for EcoRI, SalI, BamHI, HindIII and XhoI, respectively;

Fig. 6 is a construction scheme for the plasmid pTRP P31-R for the expression of subtype adr HBsAg P31 in Escherichia coli, the symbols E, B, C and P standing for EcoRI, BamHI, ClaI and PstI, respectively;

Fig. 7 is a construction scheme for the plasmid pTRP P31-W2 for the expression of subtype adw HBsAg P31 in Escherichia coli, the symbols E, B, C and P standing for EcoRI, BamHI, ClaI and PstI, respectively;

Fig. 8 is a construction schema for the plasmids pGLD P31-R, pPHO P31-R AND pPKT P31-R for the expression of subtype adr HBsAg P31 in yeast, the symbols E, B, S, H, X and C standing for EcoRI, BamHI, SalI, HindIII, XhoI and ClaI, respectively;

Fig. 9 and Fig. 10 each is a construction scheme for the plasmid pPHO P31-W for the expression of subtype adw HBsAg P31 in yeast, the symbols E, P, B, C, S and H standing for EcoRI, PstI, BamHI, ClaI, SalI and HindIII, respectively; and

Fig. 11 shows the results of SDS-polyacrylamide slab gel electrophoresis as described in paragraphs (1) and (2) in the last portion of Example 7.

Best Mode for Carrying Out the Invention

Reference Example 1

Preparation of expression vector containing yeast-derived repressible acid phosphatase promoter (PHO5-P)

The Escherichia coli plasmid pJA1 (50 μg) containing a Saccharomyces cerevisiae S288C-derived, repressible acid phosphatase gene (PHO5)- and constitutive acid phosphatase gene (PHO3)-containing, 7.9 kb DNA fragment [Kramer, R. A. and Anderson, N., Proc. Natl. Acad. Sci. USA, 77, 6541 (1980)] was treated with 20 units of the restriction enzyme BamHI [Takara Shuzo] and 20 units of the restriction enzyme SalI [Takara Shuzo] in 100 μl of a reaction mixture [10 mM Tris-HCl (pH 8.0), 7 mM $MgCl_2$, 100 mM NaCl, 2 mM 2-mercaptoethanol] at 37°C for 3 hours, followed by 1.0% agarose (Sigma) slab gel electrophoresis in a buffer [100 mM Tris-HCl, 100 mM boric acid, 2 mM EDTA (pH 8.3] at 140 V for 2 hours. Thereafter, a gel portion containing a 0.63 kb DNA fragment was sealed in a dialytic tube, the tube was immersed in a buffer for electrophoresis, and said DNA fragment was eluted electrically from the gel [McDonell, M. W. et al., J. Mol. Biol., 110, 119 (1977)]. The liquid in the dialytic tube was extracted with phenol, followed by extraction with ether and addition of NaCl to a concentration of 0.2 M. Then, two volumes of cold ethanol was added and DNA precipitation was caused at -20°C.

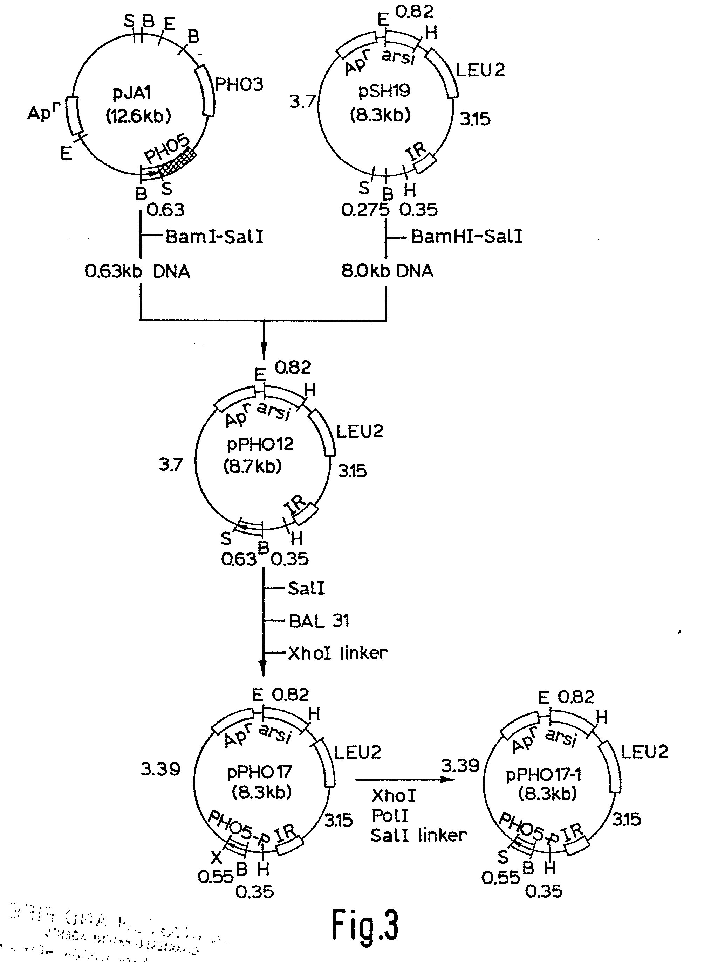

The plasmid pSH19 (1 μg) was treated with 2 units of the restriction enzyme BamHI and 2 units of the restriction enzyme SalI in 20 μl of a reaction mixture [10 mM Tris-HCl (pH 8.0), 7 mM MgCl$_2$, 100 mM NaCl, 2 mM 2-mercaptoethanol] at 37°C for 2 hours. The reaction mixture was subjected to electrophoresis using a 0.8% agarose slab gel, which was conducted under the same conditions as above. After electrophoresis, a 8.0 kb DNA fragment was isolated from the gel by the above-mentioned method. Following deproteinization with phenol, the DNA was precipitated with cold ethanol (cf. Fig. 3).

A 400-ng portion of said 8.0 kb DNA fragment and a 200-ng portion of the above 0.63 kb DNA fragment were mixed and ligated together in 20 μl of a reaction mixture [66 mM Tris-HCl (pH 7.6), 6.6 mM MgCl$_2$, 10 mM di-

thiothreitol, 1 mM ATP, 2 units of T4 DNA ligase (Takara Shuzo)] at 14°C.  After overnight reaction, the reaction mixture was used for transforming Escherichia coli 294 by the above-cited method of Cohen et al.  The plasmid DNA was isolated by the above-mentioned alkaline extraction method from each transformant selected based on ampicilline resistance as the indicator and examined for molecular weight and restriction enzyme cleavage pattern.  In this

way, a plasmid, pPHO12, in which the 0.63 kb DNA fragment isolated from pJA1 had been inserted into the BamHI-SalI site of pSH19 was separated (cf. Fig. 3).

A 3-μg portion of the plasmid pPHO12 DNA was

treated with 2 units of the restriction enzyme SalI in

20 μl of a reaction mixture [10 mM Tris-HCl (pH 7.5),

7 mM MgCl$_2$, 175 mM NaCl, 0.2 M EDTA, 7 mM 2-mercaptoethanol] at 37°C for 2 hours, followed by deproteinization with phenol and precipitation of DNA with cold

ethanol.  A 3-μg portion of this DNA was treated with 12 units of BAL31 nuclease (Bethesda Research Laboratories)

in 50 μl of a reaction mixture [20 mM Tris-HCl (pH 8.1),

12 mM CaCl$_2$, 12 mM MgCl$_2$, 1 mM EDTA] at 30°C for 2 minutes, followed by deproteinization with phenol and DNA precipitation with cold ethanol (cf. Fig. 3).  The

XhoI linker  d(CCTCGAGG) (200 ng) [New England BioLabs] was phosphorylated at the 5'-end thereof by treating with 3 units of T4 polynucleotide kinase [Takara Shuzo] in 50 μl of a

reaction mixture [50 mM Tris-HCl (pH 7.6), 10 mM MgCl$_2$,

10 mM 2-mercaptoethanol, 100 μM ATP] at 37°C for 1 hour.

A 40-ng portion of the 5'-end phosphorylated XhoI linker [5'-P-d(CCTCGAGG)] and 400 ng of the above-mentioned BAL31-treated pPHO12 DNA were mixed and ligated together under the action of T4 DNA ligase under the same conditions as mentioned above. Using this reaction mixture, Escherichia coli 294 was transformed by the method of Cohen et al. The plasmid DNA was isolated by the alkaline extraction method mentioned above from each transformant selected on the basis of ampicillin resistance as the indicator, and a plasmid, pPHO17, which gave a 0.55 fragment upon double digestion with BamHI and XhoI was selected. Analysis of the DNA base sequence by the dideoxynucleotide method [Sanger, F. et al., Proc. Natl. Acad. Sci. USA, 74, 5463 (1977)] revealed that the BAL31 nuclease treatment had resulted in elimination of 20 bp upstream from the start codon ATG in PHO5 (cf. Fig. 3).

Then, 2 μg of said plasmid pPHO17 was treated with 4 units of the restriction enzyme XhoI [Takara Shuzo] in 20 μl of a reaction mixture [6 mM Tris-HCl (pH 7.9), 150 mM NaCl, 6 mM $MgCl_2$, 5 mM 2-mercaptoethanol] at 37°C for 2 hours, followed by deproteinization with phenol and DNA precipitation with cold ethanol.

A 1-μg portion of the DNA precipitated was treated with 5 units of DNA polymerase I large fragment (New England BioLabs) in 30 μl of a reaction mixture [40 mM potassium phosphate buffer (pH 7.5), 6.6 mM $MgCl_2$, 1 mM

0171908

2-mercaptoethanol, 33 µM dATP, 33 µM dGTP, 33 µM dTTP, 33 µM dCTP] at 12°C for 30 minutes to thereby convert the cohesive ends to blunt ends, followed by deproteinization with phenol and precipitation of DNA with cold ethanol. A 500-ng portion of said DNA fragment and 50 ng of SalI linker phosphorylated under the conditions mentioned above [5'-P-d(GGTCGACC)] (New England BioLabs) were mixed and ligated together under the action of T4 DNA ligase under the conditions mentioned above. Using this reaction mixtre, Escherichia coli 294 was transformed by the method of Cohen et al. From among the ampicillin-resistant transformants, a plasmid, pPHO17-1, in which the XhoI site of the plasmid pPHO17 had been converted to a SalI site was selected (cf. Fig. 3).

Reference Example 2

Preparation of expression vector containing yeast phosphoglycerate kinase promoter (PGK-P)

(1) Cloning of phosphoglycerate kinase gene (PGK)

A 350-µg portion of the chromosomal DNA of the Saccharomyces cerevisiae strain Kyokai 3 (available from IFO) as prepared by the method of Cryer, D. R. et al. [Methods in Cell Biology, Vol. XII, pages 39-44 (1975)] was treated with 200 units of the restriction enzyme HindIII [Takara Shuzo] in 1 ml of a reaction mixture [10 mM Tris-HCl (pH 7.5), 7 mM $MgCl_2$, 60 mM NaCl] at 37°C for 3 hours, followed by 1% agarose slab gel electrophoresis under the conditions described in Reference Example 1. After electrophoresis, the agarose
gel was divided into fractions 1 to 10 depending on the size
of DNA fragment. The agarose gel piece of each fraction
was sealed in a dialytic tube and DNA was eluated electrically
from the gel piece under the conditions
described in Reference Example 1. The eluate was treated
with phenol and the DNA was precipitated by addition of
cold ethanol. A 0.5-μg portion of the DNA from each
fraction was electrophoresed under the conditions described in Reference Example 1 using a 1% agarose slab
gel and then the DNA was allowed to be adsorbed on a
nitrocellulose filter (Schleicher and Schull) by the
method of Southern [Southern, E.M., J. Mol. Biol., 98, 503 (1975)].
Oligonuckotide, 5'-TGAAGATAAAGACAT-3', complementary to
the oligonucleotide coding for 5 amino acids from the
N-terminal of PGK [Dobson, M. J. et al., Nucleic Acids
Res., 10, 2625 (1982)], was synthesized by the mehtod
of Crea, R. et al. [Proc. Natl. Acad. Sci. USA, 75,
5765 (1978)] and a 1-μg portion of said oligonucleotide
was treated with 10 μCi of γ-[$^{32}$P]ATP (Amersham) and 10
units of T4 polynucleotide kinase in 30 μl of a reaction
mixture [50 mM Tris-HCl (pH 7.6), 10 mM MgCl$_2$, 10 mM
2-mercaptoethanol] at 37°C for 30 minutes to thereby
label the 5'-end with $^{32}$P. To the reaction mixture,
there was added 10 μl of 200 mM EDTA (pH 8.0), and the
mixture was deproteinized with 1 volume of phenol and
then applied to a Sepharose 4B (Pharmacia) column (0.25
x 25 cm) equilibrated with TEN buffer [10 mM Tris-HCl (pH 8.0), 200 mM NaCl, 1 mM EDTA]. The labelled oligonucleotide eluted in the vicinity of void volume was collected and used as the probe for screening for the PGK gene. Blotting was performed by the above-mentioned method of Southern using the above-mentioned nitrocellulose filter and said probe, whereupon the probe hybridized strongly with the sample of fraction No. 3 containing 2.6 kb to 2.9 kb DNA fragments.

Then, 10 μg of the cloning vector pTR262 [Roberts, T. M. et al., Gene, 12, 123 (1980)] was treated with 10 units of the restriction enzyme HindIII in 50 μl of a reaction mixture [10 mM Tris-HCl (pH 7.5), 7 mM MgCl$_2$, 60 mM NaCl] at 37°C for 2 hours, followed by deproteinization with phenol and DNA precipitation with cold ethanol (HindIII-digested pTR262). A 0.1-μg portion of the HindIII-digested pTR262 was mixed with 0.2 μg of the DNA of fraction No. 3 and they are ligated together under the action of T4 DNA ligase under the conditions mentioned in Reference Example 1. Using the reaction mixture, Escherichia coli DH1 [Maniatis, T. et al., Molecular Cloning, Cold Spring Harbor Laboratory, 254-255 (1982)] was transformed, and there were obtained about 1,300 transformants showing tetracycline resistance. From among them, there was selected a PGK gene-carrying transformant was selected by colony hybridization [Suggs, S. V. et al., Proc. Natl. Acad. Sci. USA, 78, 6613 (1981)] using the above-mentioned $^{32}$P-labelled synthetic probe.

From a tranformant which gave a strong signal in autoradiography, a plasmid, pPKT3, was isolated by the above-mentioned alkaline extraction method.  Digestion with HindIII led to detection of a 2.95 kb DNA insert and test by the method of Southern confirmed that said insert hybridized with said probe.

(2)   Isolation of PGK promoter fragment

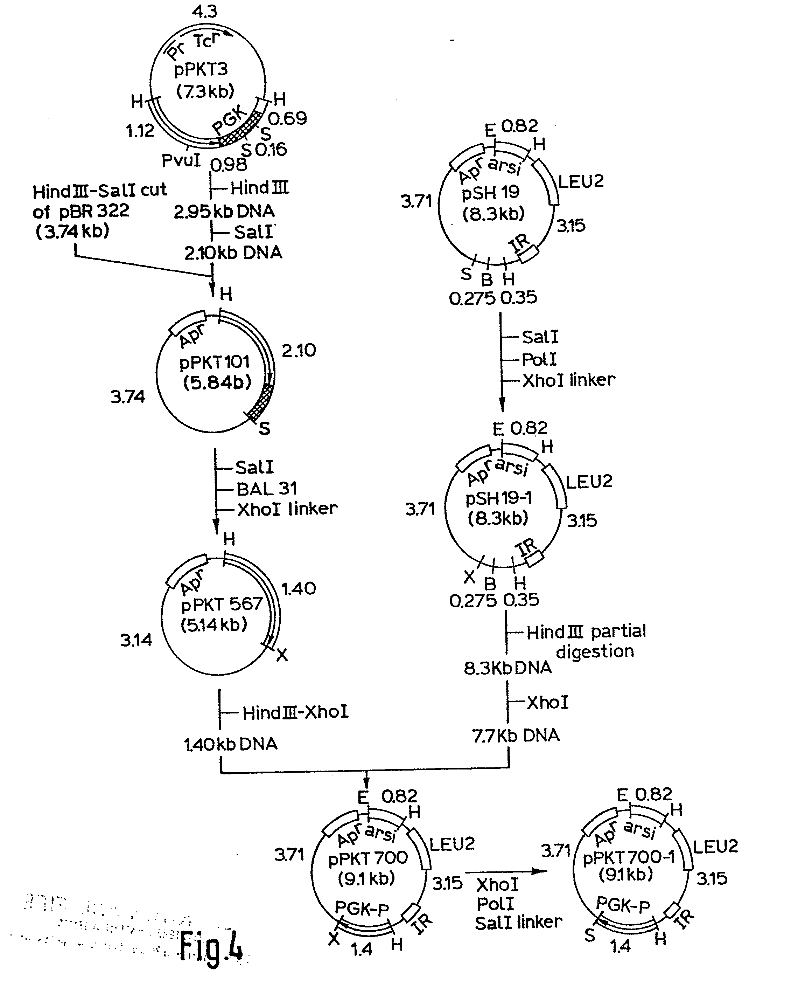

A 50-μg portion of the plasmid pPKT3 DNA was treated with 50 units of the restriction enzyme HindIII in 100 μl of a reaction mixture [10 mM Tris-HCl (pH 7.5), 7 mM MgCl$_2$, 60 mM NaCl] at 37°C for 2 hours, followed by 1% agarose slab gel electrophoresis under the conditions described in Refernce Example 1.  After electrophoresis, the 2.95 kb DNA fragment was separated from the gel by the method described in Reference Example 1 (cf. Fig. 4).

A 5-μg portion of said 2.95 kb DNA fragment was treated with 5 units of the restriction enzyme SalI in 20 μl of a reaction mixture [10 mM Tris-HCl (pH 7.5), 7 mM MgCl$_2$, 175 mM NaCl, 7 mM 2-mercaptoethanol] at 37°C for 3 hours and the digestion mixture was subjected to electrophoresis using a 1.2% agarose slab gel, which was conducted under the conditions described in Reference Example 1.  After electrophoresis, a 2.1 kb DNA fragment was separated from the gel by the method described in Reference Example 1.  A 0.5-μg portion of said 2.1 kb DNA fragment and 0.5 μg of a 3.74 kb DNA obtained by digestion of the plasmid pBR322 with HindIII and SalI were mixed and ligated together under the action of T4 DNA ligase under the conditions described in Reference Example 1.   Using the reaction mixture, Escherichia coli DH1 was transformed, and a desired plasmid, pPKT101, was obtained  from among the ampicilline-resistant transformants obtained (cf. Fig. 4).

Then, for eliminating the structural gene region of the PGK gene, a 10-µg portion of said plasmid pPKT101 DNA was first treated with 10 units of the restriction enzyme SalI in 30 µl of a reaction mixture [10 mM Tris-HCl (pH 7.5), 7 mM $MgCl_2$, 175 mM NaCl, 0.2 M EDTA, 7 mM 2-mercaptoethanol] at 37°C for 3 hours, the digestion mixture was deproteinized with phenol, and DNA precipitation was caused with cold ethanol (SalI-digested pPTK101).

A 1-µg portion of the SalI-digested pPTK101 was then treated with 10 units of BAL31 nuclease in 20 µl of a

reaction mixtrue [20 mM Tris-HCl (pH 8.1), 12 mM $CaCl_2$,

12 mM $MgCl_2$, 1 mM EDTA] at room temperature for 5 minutes.

Immediately thereafter, 1 volume of phenol was added to terminate the reaction, followed by DNA precipitation

with cold ethanol (BAL-digested pPTK101).  A 50-ng portion

of the phosphorylated XhoI linker described in Reference Example 1 and a 0.2-µg portion of BAL-digested pPTK101 were mixed and ligated together using T4 DNA ligase under the conditions described in Reference Example 1.  Thereafter, Escherichia coli DH1 was transformed using the reaction mixtrue and, from among the ampicillin-resistant transformants obtained, a plasmid, pPKT567, with 0.69

kb from the SalI site of pPKT101 eliminated in the direction of the promoter region, was obtained. Analysis of the DNA base sequence by the dideoxynucleotide method proved that, in pPKT567, the BAL31 treatment had eliminated the PGK structural gene and up to 5'-vicinity region-24 (cf. Fig. 4).

(3)   Construction of expression vector

A 5-µg portion of the Escherichia coli-yeast shuttle vector pSH19 was treated with 6 units of the restriction enzyme SalI in 20 µl of a reaction mixture [10 mM Tris-HCl (pH 7.5), 7 mM MgCl$_2$, 175 mM NaCl, 0.2 mM EDTA, 7 mM 2-mercaptoethanol] at 37°C for 2 hours, followed by deproteinization with phenol and precipitation with cold ethanol.   A 1-µg portion of the DNA was treated with DNA polymerase I large fragment under the conditions as mentioned in Reference Example 1 were mixed and ligated cohesive end to a blunt end.   A 500-ng portion of said DNA fragment and 50 ng of the phosphorylated XhoI linker as mentioned in Referance Example 1 were mixed and ligated together using T4 DNA ligase under the conditions described in Reference Example 1.   Escherichia coli DH1 was transformed with the reaction mixture and, from among the ampicilin-resistant transformants obtained, a transformant carrying a plasmid, pSH19-1, in which the SalI site of pSH19 had been converted to a XhoI site was obtained (cf. Fig. 4).

A 15-µg portion of said plasmid pSH19-1 DNA was treated with 24 units of the restriction enzyme HindIII in 100 µl of a reaction mixture [10 mM Tris-HCl (pH 7.5),

7 mM MgCl$_2$, 60 mM NaCl] at 37°C for 10 minutes. Immediately thereafter, the reaction was terminated by adding 10 μl of 0.2 M EDTA. The reaction mixture was subjected to electrophoresis using a 0.7% agarose slab gel under the conditions described in Reference Example 1, and a 8.3 kb DNA fragment cleaved at one site with HindIII was separated from the gel by the method described in Reference Example 1. A 3-μg portion of said 8.3 kb DNA fragment was treated with 10 units of the restriction enzyme XhoI [Takara Shuzo] in 30 μl of a reaction mixtrue [10 mM Tris-HCl (pH 7.5), 7 mM MgCl$_2$, 100 mM NaCl, 7 mM 2-mercaptoethanol] at 37°C for 2 hours. The reaction mixture was electrophoresed using a 0.7% agarose slab gel under the conditions described in Reference Example 1. After electrophoresis, a 7.7 kb DNA fragment was separated from the gel by the method described in Reference Example 1 (cf. Fig. 4).

A 10-μg portion of the plasmid pPKT567 DNA described in Reference Example 2-(2) was treated with 10 units each of the restriction enzymes HindIII and XhoI in 50 μl of a reaction mixture [50 mM Tris-HCl (pH 7.6), 50 mM NaCl, 1 mM dithiothreitol, 10 mM MgCl$_2$] at 37°C for 2 hours. Thereafter, electrophoresis was performed using a 1.2% agarose slab gel under the conditions described in Reference Example 1, and a 1.40 kb DNA fragment was separated from the gel (cf. Fig. 4).

A 0.2-μg portion of said 1.40 kb DNA fragment and 0.5 µg of the above-mentioned 7.7 kb DNA fragment were mixed and ligated together using T4 DNA ligase under the conditions described in Reference Example 1.  Escherichia coli DH 1 was transformed with the reaction mixture and, from among the ampicillin-resistant transformants obtained, there was  obtained a transformant carrying the desired plasmid pPKT700.  Then, a plasmid, pPKT700-1, with the XhoI site of said plasmid pPKT700 converted to a SalI site was constructed by the method described in Reference Example 1 (cf. Fig. 4).

Reference Example 3

    Construction of expression vector containing
    yeast glyceraldehyde-3-phosphate dehydrogenase
    promoter (GLD-P)

(1)  Cloning of glyceraldehyde-3-phosphate dehydrogenase
     gene (GLD)

    5'-AGCAACTCTAACCAT-3', complementary to that oligonucleotide of pgap491 [Holland, J. P. et al., J. Biol. Chem., 258, 5291 (1983)] of GLD which codes for the 5 amino acids from the N-terminal, was synthesized by the above-mentioned method of Crea, R. et al., labelled with $^{32}$P by the method described in Reference Example 2-(1), and used as the probe.  Southern blotting was performed using the nitrocellulose filter described in Reference Example 2-(1) and said probe, whereupon the probe strongly hybridized with the sample of fraction No. 7 containing 2.0-2.3 kb DNA fragments.

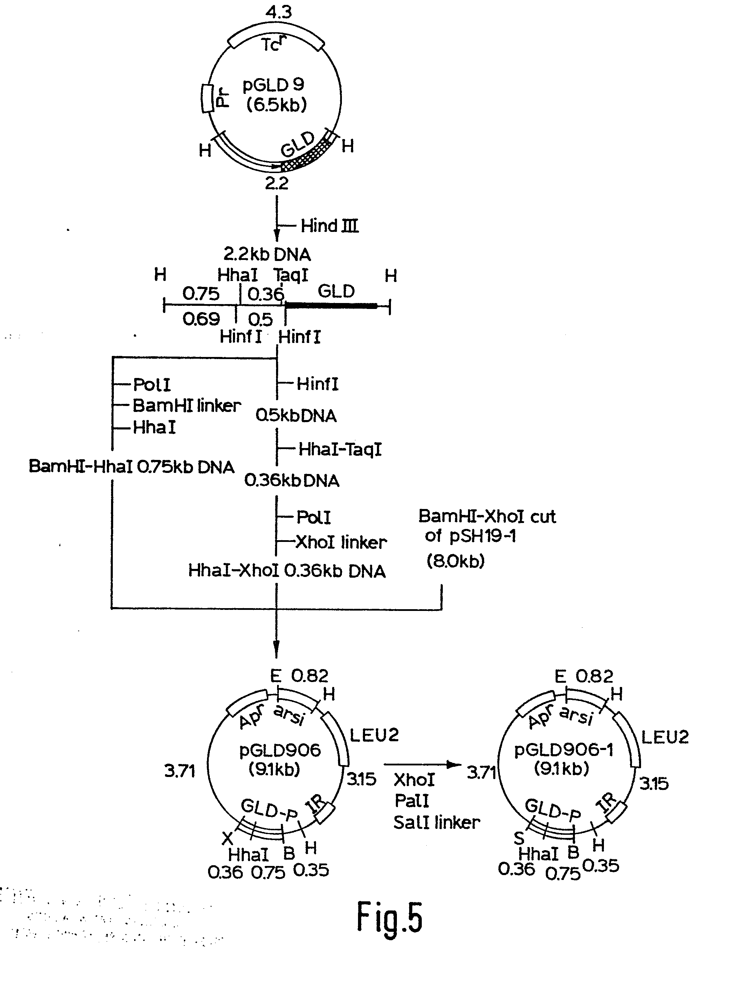

0.1 µg of the HindIII-digested pTR262 described in Reference Example 2-(1) and 0.2 µg of the DNA of fraction No. 7 were mixed and ligated together using T4 DNA ligase under the conditions described in Reference Example 1. Using the reaction mixture, Escherichia coli DH1 was transformed by the method described in Reference Example 2-(1) and, from among about 1,200 tetracycline-resistant transformants obtained, a transformant capable of hybridizing strongly with the $^{32}$P-labelled probe was isolated by colony hybridization. A plasmid, pGLD9, was isolated from this transformant by the above-mentioned alkaline extraction method. Upon digestion with HindIII, a 2.2 kb insert DNA was detected, and test by the method of Southern confirmed that this insert DNA hybridized with said probe (cf. Fig. 5).

(2)  Isolation of GLD promoter fragment

100 µg of the plasmid pGLD9 DNA was treated with 50 units of the restriction enzyme HindIII in 200 µl of a reaction mixture [10 mM Tris-HCl (pH 7.5), 7 mM MgCl$_2$, 60 mM NaCl] at 37°C for 3 hours, followed by electrophoresis using a 1.0% agarose slab gel under the conditions described in Reference Example 1. After electrophoresis, the 2.2 kb DNA fragment was separated from the gel by the method described in Reference Example 1. 10 µg of said 2.2 kb DNA fragment was treated with 10 units of the restriction enzyme HinfI [Takara Shuzo] in 50 µl of a reaction mixture [10 mM Tris-HCl (pH 7.5), 7 mM MgCl$_2$, 100 mM NaCl, 7 mM 2-mercaptoethanol] at 37°C for 2 hours and then hybridization was performed by the method of Southern using the probe for GLD, whereupon said probe was bound to a 0.5 kb DNA fragment (cf. Fig. 5).

5 μg of said 0.5 kb DNA fragment was treated with 10 units each of the restriction enzymes HhaI [Takara Shuzo] and TaqI (New England BioLabs) in 30 μl of a reaction mixture [10 mM Tris-HCl (pH 7.5), 50 mM NaCl, 10 mM MgCl$_2$, 1 mM dithiothreitol] at 37°C for 3 hours, followed by 1.5% agarose slab gel electrophoresis, which was conducted under the conditions described in Reference Example 1. Thereafter, a 0.36 kb DNA fragment was separated from the gel by the method described in Reference Example 1 (cf. Fig. 5).

1 μg of said 0.36 kb DNA fragment was treated with DNA polymerase I large fragment under the conditions described in Reference Example 1 to thereby render the TaqI cohesive end blunt. Then, 1 μg of this fragment and 50 ng of the phosphorylated XhoI linker described in Reference Example 1 were mixed and ligated together using T4 DNA ligase under the conditions described in Reference Example 1. After reaction, XhoI was added in excess and, after 4 hours of reaction at 37°C, a linker-bound 0.36 kb DNA fragment was separated using a Sepharose 4B column under the conditions described in Reference Example 2-(1).

Separately, 10 μg of the above-mentioned 2.2 kb DNA fragment was treated with DNA polymerase I large fragment under the conditions described in Reference Example 1 to render the cohesive end blunt and thereafter ligated with 50 ng of the phosphorylated BamHI linker [5'-P-d(CGCGGATCCGCG)] (new England BioLabs) described in Reference Example 1 using T4 DNA ligase under the conditions described in Reference Example 1. After reaction, 20 units of BamHI was added and, after 3 hours of reaction at 37°C, a linker-bound 2.2. kb DNA fragment was separated using a Sepharose 4B column under the conditions described in Reference Example 2-(1). 6 µg of said DNA fragment was treated with 2 units of the restriction enzyme HhaI in 50 µl of a reaction mixture [10 mM Tris-HCl (pH 7.5), 50 mM NaCl, 10 mM MgCl$_2$, 1 mM dithiothreitol] at 37°C for 2 hours. Thereafter, the mixture was electrophoresed using a 1.0% agarose slab gel under the conditions described in Reference Example 1. After electrophoresis, a 0.75 kb DNA fragment was separated from the gel by the method described in Reference Example 1 (cf. Fig. 5).

(3)  Construction of expression vector

10 µg of the plasmid pSH19-1 described in Reference Example 2-(3) was treated with 10 units each of the restriction enzymes BamHI and XhoI in 50 µl of a reaction mixture [10 mM Tris-HCl (pH 7.5), 7 mM MgCl$_2$, 100 mM NaCl, 7 mM 2-mercaptoethanol] at 37°C for 2 hours, followed by 1.0% agarose slab gel electrophoresis under the conditions described in Reference Example 1. Thereafter, an 8.0 kb DNA fragment was separated from the gel by the method described in Reference Example 1.

500 ng of said 8.0 kb DNA fragment, 200 ng of the 0.36 kb DNA fragment described in Reference Example 3-(2) and 200 ng of the 0.75 kb DNA fragment described in
Reference Example 3-(2) were mixed and ligated together
using T4 DNA ligase under the conditions described in
Reference Example 1. Using the reaction mixture,
Escherichia coli DH 1 was transformed and, from among the
ampicillin-resistant transformants obtained, a transformant carrying a plasmid, pGLD906, composed of the
three DNA fragments ligated together was separated.
Then, a plasmid, pGLD906-1, was constructed by converting
the XhoI site of said plasmid pGLD906 to a SalI site
(cf. Fig. 5).

Reference Example 4

    Production of poly-HSA-Cellulofine column

(1) Production of polymerized human serum albumin

    To 20 ml of a human serum albumin (Albumin-Nichiyaku;
Nippon Seiyaku, Tokyo) solution (containing 4 g of human
serum albumin), there were added 180 ml of phosphate-
buffered saline (PBS; 8.1 mM $NaH_2PO_4$, 1.5 mM $KH_2PO_4$,
2.7 mM KCl, 137 mM NaCl, pH 7.2) and 60 ml of 2% aqueous
glutaraldehyde. The mixture was stirred to attain
homogeneity and then allowed to stand at room temperature
for 1 hour. The polymerization reaction was then terminated by dialyzing the above mixed solution against 10
liters of distilled water at 5°C. Thereafter, the
dialyzing fluid was exchanged three times so that
the reamining glutaraldehyde was completely removed
from the reaction mixture. There was thus obtained 275
ml of a polymerized human serum albumin (poly-HSA) solution.

(2)  Production of poly-HSA-Cellulofine column

To 90 ml of the poly-HSA solution obtained in the above manner, there was added 10 ml of  10 times concentrated PBS, followed by addition of 40 ml of Formyl-Cellulofine.  After stirring, the reaction was allowed to proceed at room temperature for 30 minutes. Then, 1.6 g of NaCNBH$_3$ was added to that mixture and, after tight stoppering, the whole mixture was shaken at room temperature for 18 hours.  The mixture was transferred onto a glass filter and the gel was washed with PBS to thereby remove that portion of poly-HSA that remained unbound to the Formyl-Cellulofine.  The percentage binding of poly-HSA to Cellulofine in the above procedure was about 73%, and there was obtained 40 ml of poly-HSA-Cellulofine containing about 24 mg of poly-HSA bound to each ml of Cellulofine.  This gel was suspended in PBS containing 0.1 M ethanolamine, NaCNBH$_3$ (200 mg) was added and the mixture was shaken at room temperature for 3 hours, and the gel was then transferred onto a glass filter, washed in sequence with PBS, 3 M urea, 6 M guanidine hydrochloride and 25 mM sodium phosphate buffer (pH 7.5) and packed into a column having an inside diameter of 5 cm, giving a poly-HSA-Cellulofine column.

Example 1

Construction of recombinant DNA molecule for expression of subtype adr hepatitis B virus surface antigen P31 gene and transformation of Escherichia coli

with said DNA molecule

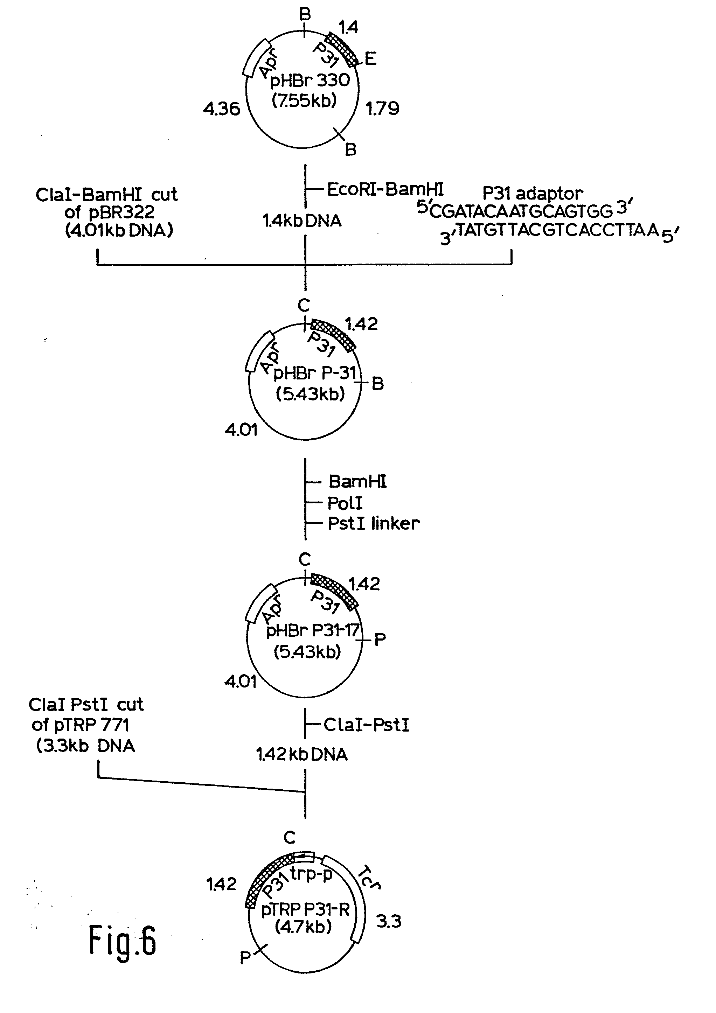

The plasmid pBR322-BamHI/HBr330 DNA (also referred
to as pHBr330 for short) as mentioned in Japanese published
unexamined patent application No. 74985/1984 and in
Nucleic Acids Res., 11, 1747 (1983) was prepared by the
method described in Japanese published unexamined patent
application No. 201796/1983 in Reference Example 1 thereof.
50 μg of said plasmid pHBr330 was treated with 20 units
each of the restriction enzymes EcoRI [Takara Shuzo] and
BamHI in 100 μl of a reaction mixture [100 mM Tris-HCl
(pH 7.5), 7 mM MgCl$_2$, 50 mM NaCl, 7 mM 2-mercaptoethanol]
at 37°C for 3 hours, followed by electrophoresis using
a 1.0% agarose slab gel under the conditions described
in Reference Example 1. After electrophoresis, a 1.4 kb
DNA fragment was separated from the gel by the method
described in Reference Example 1 (cf. Fig. 6).

2 μg of the plasmid pBR322 DNA was treated with
2 units each of the restriction enzymes BamHI and ClaI
(New England BioLabs) in 20 μl of a reaction mixture
[10 mM Tris-HCl (pH 8.0), 7 mM MgCl$_2$, 100 mM NaCl, 2 mM
2-mercaptoethanol] at 37°C for 2 hours, followed by 0.8%
agarose slab gel electrophoresis, which was conducted
under the conditions described in Reference Example 1.
Thereafter, a 4.01 kb DNA fragment was separated from
the gel by the method described in Reference Example 1.

500 ng of the above 4.01 kb DNA fragment, 500 ng
of the above 1.4 kb DNA fragment and 50 ng of a
synthetic adapter d($_{3'}^{5'} {}^{CGATACAATGCAGTGG}_{TATGTTACGTCACCTTAA}{}^{3'}_{5'}$)

phosphorylated at the 5'-end by the method described in Reference Example 1 were ligated together under the conditions described in Reference Example 1 using T4 DNA ligase. The above-mentioned adapter was chemically synthesized by the phosphotriester method [Crea, R. et al., Proc. Natl. Acad. Sci. USA, 75, 5765 (1978)]. Using the above reaction mixture, Escherichia coli 294 was transformed and, from among the ampicillin-resistant transformants obtained, a plasmid pHBrP31 DNA composed of the above three DNAs ligated together was obtained (cf. Fig. 6).

1 μg of the plasmid pHBrP31 DNA was treated with 2 units of the restriction enzyme BamHI in 20 μl of a reaction mixture [10 mM Tris-HCl (pH 8.0), 7 mM MgCl$_2$, 100 mM NaCl, 2 mM 2-mercaptoethanol] at 37°C for 2 hours, followed by deproteinization with phenol and DNA precipitation by addition of cold ethanol (BamHI-digested pHBrP31).

500 ng of the BamHI-digested pHBrP31 was treated with DNA polymerase I large fragment under the conditions described in Reference Example 1 to render the cohesive ends blunt, followed by deproteinization with phenol and DNA precipitation with cold ethanol. 300 ng of the DNA fragment obtained and 50 ng of a PstI linker phosphorylated at the 5'-end by the method described in Reference Example 1 [5'-P-d(GCTGCAGC)] (New England BioLabs) were ligated together under the conditions described in Reference Example 1 using T4 DNA ligase. Using the reaction mixture, Escherichia coli 294 was transformed,    the transformants obtained were screened by the method described in Reference Example 1 and a plasmid, pHBrP31-17, wherein the BamHI site of the plasmid pHBrP31 had been converted to a PstI site, was separated (cf. Fig. 6).

50 µg of said pHBrP31-17 was treated with 20 units each of the restriction enzymes ClaI and PstI [Takara Shuzo] in 100 µl of a reaction mixture [20 mM Tris-HCl (pH 7.5), 10 mM $MgCl_2$, 50 mM $(NH_4)_2SO_4$] at 37°C fpr 3 hours, followed by electrophoresis using a 1.0% agarose slab gel under the conditions described in Reference Example 1.  After electrophoresis, a 1.42 kb DNA fragment was separated from the gel by the method described in Reference Example 1.

50 µg of the expression vector pTRP771 described in Japanese published unexamined patent application No. 201796/1983 and in Nucleic Acids Res., 11, 3581 (1983) was treated with the restriction enzymes ClaI and PstI in 100 µl of the above reaction mixture under the same conditions and then the reaction mixture was subjected to electrophoresis using a 1.0% agarose slab gel under the conditions described in Reference Example 1.  After electrophoresis, a 3.3 kb DNA fragment was  separated from the gel by the method described in Reference Example 1.

200 ng of said 1.42 kb DNA (DNA coding for P31) and 500 ng of the 3.3 kb DNA were ligated together under the conditions described in Reference Example 1 using T4 DNA ligase. Said reaction mixture was used for transforming Escherichia coli 294 and an Escherichia coli strain (294/pTRP P31-R) carrying a plasmid, pTRP P31-R, in which said P31-encoding DNA was inserted into the expression vector was isolated by the method of Reference Example 1 (cf. Fig. 6).

Example 2

Construction of recombinant DNA molecule for expression of subtype adw hepatitis B virus surface antigen P31 gene and transformation of Escherichia coli with said DNA molecule

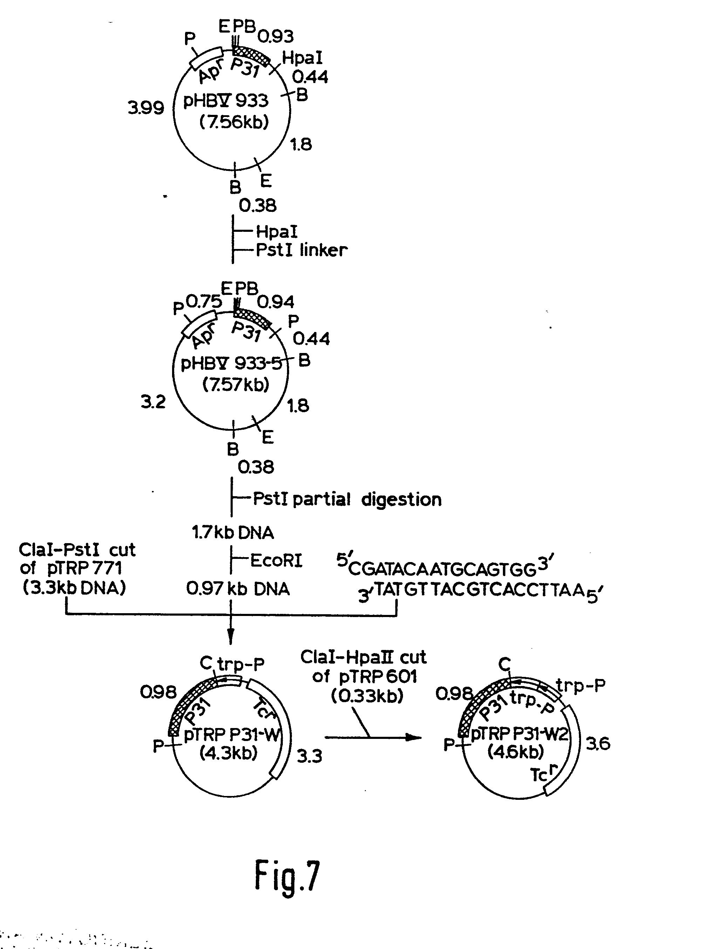

The plasmid pBR-EcoRI/HBV933 DNA (referred to as pHBV933 for short) described in Japanese published unexamined patent applications Nos. 194897/1983 and 201796/1983 and in Nucleic Acids Res., 11, 1747 (1983) was prepared by the method described in Japanese published unexamined patent application No. 201796/1983 in Reference Example 1 thereof. 2 μg of said plasmid was treated with 2 units of the restriction enzyme HpaI [Takara Shuzo] in 20 µl of a reaction mixture [10 mM Tris-HCl (pH 7.5), 7 mM $MgCl_2$, 100 mM KCl, 7 mM 2-mercaptoethanol] at 37°C for 2 hours, followed by deproteinization with phenol and precipitation of a DNA with cold ethanol. 300 ng of said DNA and 50 ng of a phosphorylated PstI linker [5'-P-(GCTGCAGC)] were ligated together under the conditions described in Reference Example 1. Escherichia coli 294 was transformed using the reaction mixture, and the transformants obtained were screened by the method described in Reference Example 1, and a plasmid, pHBV933-5, in which the HpaI site of the plasmid pHBV933 had been converted to a PstI site was obtained (cf. Fig. 7).

500 µg of said plasmid pHBV933-5 DNA was treated with 500 units of the restriction enzyme PstI in 800 µl of a reaction mixture [20 mM Tris-HCl (pH 7.5), 10 mM $MgCl_2$, 50 mM $(NH_4)_2SO_4$] at 37°C for 20 minutes, immediately followed by deproteinization with phenol. Said reaction product was subjected to electrophoresis under the conditions described in Reference Example 1 using a 1.0% agarose slab gel. Thereafter, a 1.7 kb DNA fragment - a PstI partial digest - was separated from the gel by the method described in Reference Example 1 (cf. Fig. 7).

3 µg of said 1.7 kb DNA was treated with 6 units of the restriction enzyme EcoRI in 20 µl of a reaction mixture [100 mM Tris-HCl (pH 7.5), 7 mM $MgCl_2$, 50 mM NaCl, 7 mM 2-mercaptoethanol] at 37°C for 1 hour, followed by electrophoresis using a 1.0% agarose slab gel, which was conducted under the conditions described in Reference Example 1. Thereafter, a 0.97 kb DNA fragment was separated from the gel by the method described in Reference Example 1 (cf. Fig. 7).

500 ng of said 0.97 kb DNA fragment, 500 ng of the 3.3 kb DNA (ClaI-PstI digest of pTRP771) described in Example 1 and 50 ng of the phosphorylated adapter

$$d \left( \begin{array}{l} 5' \text{CGATACAATGCAGTGG} 3' \\ 3' \text{TATGTTACGTCACCTTAA} 5' \end{array} \right)$$ described in Example 1 were ligated together under the conditions described in Refernce Example 1 using T4 DNA ligase. The reaction mixture was used for transforming Escherichia coli 294 and, from a tetracycline-resistant transformant obtained, a plasmid, pTRP P31-W, composed of the above three DNAs ligated together was separated by the method described in Reference Example 1. By inserting, into said plasmid at the ClaI site, a trp promoter-containing fragment of about 330 bp obtained by digesting the plasmid pTRP601 described in Japanese published unexamined patent application No. 201796/1983 with ClaI and HpaII [Takara Shuzo], a plasmid, pTRP P31-W2 (cf. Fig. 7), was completed.

Example 3

Construction of recombinant DNA molecule for expression, in yeast, of subtype adr hepatitis B virus surface antigen P31 gene and transformation of yeast with said DNA molecule

(1)    50 µg of the plasmid pHBrP31 DNA described in Example 1 was treated with 20 units each of the restriction enzymes ClaI and BamHI in 100 µl of a reaction mixture [100 mM Tris-HCl (pH 8.0), 7 mM MgCl$_2$, 100 mM NaCl, 2 mM 2-mercaptoethanol] at 37°C for 3 hours, and the reaction mixture was subjected to 1.0% agarose slab gel electrophoresis under the conditions described in Reference Example 1. After electrophoresis, a 1.42 kb DNA fragment was separated from the gel by the method described in Reference Example 1.

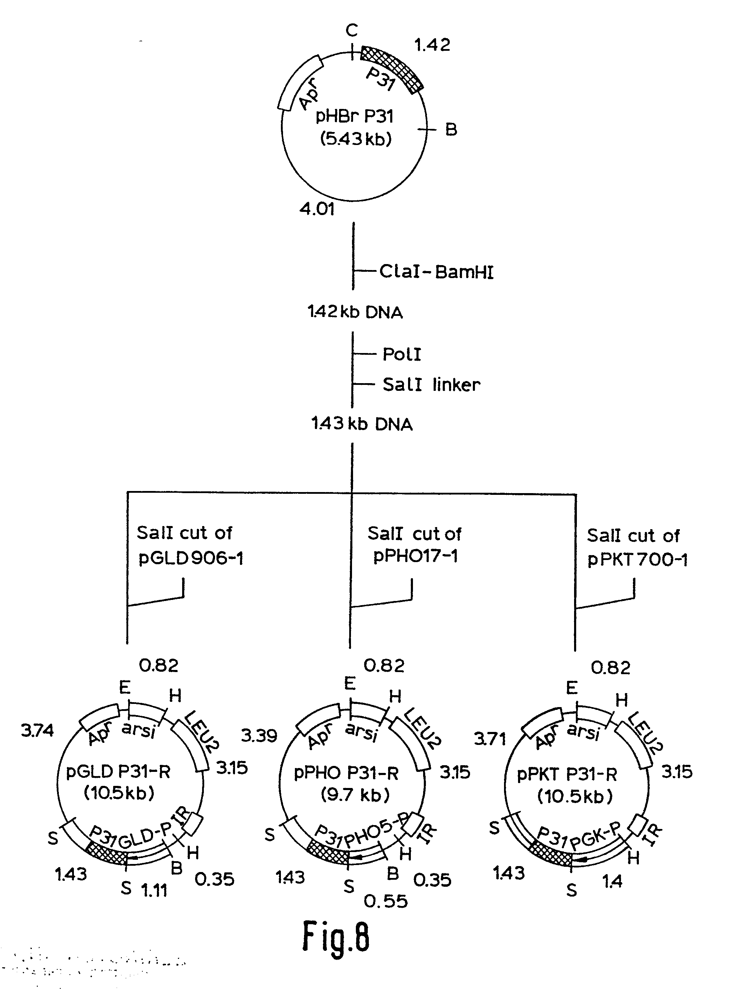

2 µg of said 1.42 kb DNA fragment was treated with DNA polymerase I large fragment under the conditions described in Reference Example 1 to render the cohesive ends blunt, followed by deproteinization with phenol and precipitation of a DNA with cold ethanol. 1.5 µg of said DNA and 50 ng of the phosphorylated SalI linker described in Reference Example 1 were ligated together under the conditions described in Reference Example 1 using T4 DNA ligase. 10 units of the restriction enzyme SalI was added to the reaction mixture, and the reaction was conducted at 37°C for 3 hours to render the ends cohesive. After reaction, deproteinization with phenol was performed and the sample was subjected to a Sepharose 4B column under the conditions described in Reference Example 2-(1). A 1.43 kb DNA fragment (subtype adr P31-encoding DNA)-containing fraction eluted in the vicinity of void volume was collected and said DNA was precipitated with cold ethanol (cf. Fig. 8).

1 µg of the expression vector pPHO17-1 DNA described in Reference Example 1 was treated with 2 units of the restriction enzyme SalI in 20 µl of a reaction mixture [6 mM Tris-HCl (pH 7.5), 6 mM MgCl$_2$, 150 mM NaCl, 6 mM 2-mercaptoethanol] at 37°C for 2 hours and, then, following addition of 0.1 unit of alkaline phosphatase, the reaction was continued at 65°C for 30 minutes. Thereafter, deproteinization with phenol was conducted and a DNA was precipitated by adding cold ethanol (SalI-digested pPHO17-1).

Then, 200 ng of the above 1.43 kb DNA fragment and 200 ng of the SalI-digested pPHO17-1 were ligated together under the conditions described in Reference Example 1 using T4 DNA ligase. Using the reaction mixture, Escherichia coli 294 was transformed, the transformants obtained were screened by the method described in Reference Example 1, and a transformant (Escherichia coli 294/pPHO P31-R) carrying a plasmid, pPHO P31-R, in which the 1.43 kb DNA fragment containing a subtype adr P31-encoding DNA had been inserted in the same directionality as the PHO 5 promoter was isolated. Said plasmid separated from this transformant by the alkaline extraction method was used for transforming the host yeast Saccharomyces cerevisiae AH22R⁻ using the method of Hinnen et al. mentioned above, and a transformant yeast (AH22R⁻/pPHO P31-R) carrying said plasmid was isolated (cf. Fig. 8).

(2)   1 µg of the expression vector pPKT700-1 DNA described in Reference Example 2-(3) is treated with 2 units of the restriction enzyme SalI at 37°C for 2 hours and then further treated with 0.1 unit of alkaline phosphatase under the conditions described in Example 3-(1). Thereafter, a DNA is precipitated with cold ethanol (SalI-digested pPKT700-1).

Then, 200 ng of the 1.43 kb DNA fragment described in Example 3-(1) and 200 ng of the SalI-digested pPKT700-1 are  ligated together under the conditions described in Reference Example 1 using T4 DNA ligase.  Using the reaction mixture and following the procedure described in Example 3-(1), a strain (294/pPKT P31-R) carrying a plasmid, pPKT P31-R, with the 1.43 kb DNA fragment containing a subtype adr P31-encoding DNA inserted therein in the same directionality as the PGK promoter is isolated.

The host yeast strain K33-8D is transformed with said plasmid, and a yeast transformant (K33-8D/pPKT P31-R) is isolated (cf. Fig. 8).

(3)    1 µg of the expression vector pGLD906-1 DNA described in Reference Example 3-(3) is treated with 2 units of the restriction enzyme SalI at 37°C for 2 hours and then further treated with 0.1 unit of alkaline phosphatase under the conditions described in Example 3-(1). Thereafter, a DNA is precipitated with cold ethanol (SalI-digested pGLD906-1).

Then, 200 ng of the 1.43 kb DNA fragment described in Example 3-(1) and the SalI-digested pGLD906-1 are ligated together under the conditions described in Reference Example 1 using T4 DNA ligase. By the method described in Example 3-(1) using the reaction mixture, a strain (294/pGLD P31-R) carrying a plasmid, pGLD P31-R, with the 1.43 kb DNA fragment containing a subtype adr P31-encoding DNA inserted therein in the same directionality as the GLD promoter is isolated. The host yeast K33-7B is transformed with said plasmid, and a yeast transformant (K33-7B/pGLD P31-R) is obtained (cf. Fig. 8).

When these transformants are cultivated by a conventional method, the desired P31 can be obtained.

Example 4

Construction of recombinant DNA molecule for expression in yeast of subtype adw hepatitis B virus surface antigen P31 gene and transformation of yeast with said DNA molecule

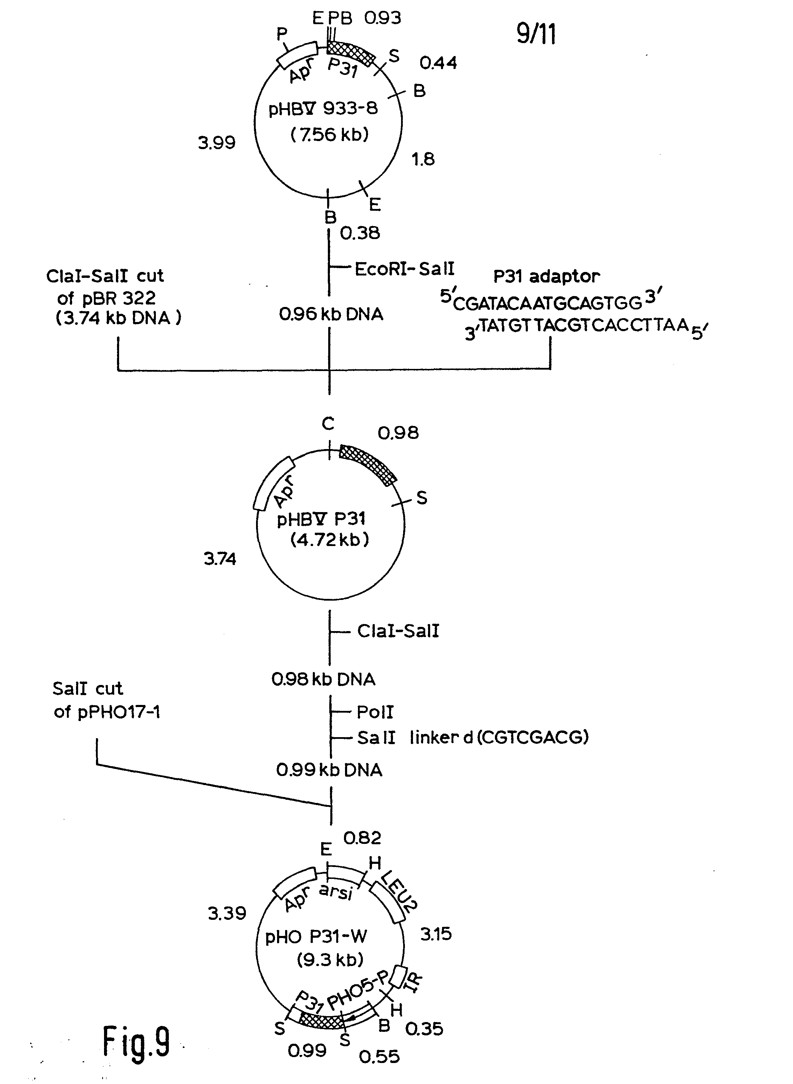

(1)    2 µg of the plasmid pHBV933 DNA described in
Example 2 was treated with 2 units of the restriction
enzyme HpaI in 20 µl of a reaction mixture [10 mM Tris-
HCl (pH 7.5), 7 mM MgCl$_2$, 100 mM KCl, 7 mM 2-mercaptoethanol]
at 37°C for 2 hours, followed by deproteinization with
phenol and precipitation of a DNA with cold ethanol.
300 ng of said DNA and 50 ng of the phosphorylated SalI
linker described in Reference Example 1 were mixed and
ligated together under the conditions described in
Reference Example 1 using T4 DNA ligase.  The reaction
mixture was used for transforming Escherichia coli 294
and, from among the ampicillin-resistant transformants
obtained, a plasmid, pHBV933-8, having a SalI site in
lieu of the HpaI site of the plasmid pHBV933 was obtained (cf. Fig. 9).

100 µg of said plasmid pHBV933-8 was treated with
100 units each of the restriction enzymes EcoRI and
SalI in 200 µl of a reaction mixture [100 mM Tris-HCl
(pH 7.5), 7 mM MgCl$_2$, 50 mM NaCl, 7 mM 2-mercaptoethanol]
at 37°C for 2 hours, and the reaction mixture was
subjected to electrophoresis under the conditions
described in Reference Example 1 using a 1.2% agarose
slab gel.  After electrophoresis, a 0.96 kb DNA fragment containing a subtype adw P31-encoding DNA fragment was separated from the gel by the method described
in Reference Example 1 (cf. Fig. 9).

20 µg of the plasmid pBR322 DNA was treated with 20 units each of the restriction enzymes ClaI and SalI in 100 µl of a reaction mixture [6 mM Tris-HCl (pH 7.9), 150 mM NaCl, 6 mM MgCl$_2$, 6 mM 2-mercaptoethanol] at 37°C for 2 hours, followed by electrophoresis using a 1.0% agarose slab gel, which was conducted under the conditions described in Reference Example 1. Thereafter, a 3.74 kb DNA fragment was separated from the gel by the method described in Reference Example 1.

500 ng of said 3.74 kb DNA fragment, 500 ng of the above-mentioned 0.96 kb DNA fragment and 50 ng of the phosphorylated adapter d ($^{5'}$CGATACAATGCAGTGG$^{3'}$ / $_{3'}$TATGTTACGTCACCTTAA$_{5'}$) described in Example 1 were ligated together under the conditions described in Reference Example 1 using T4 DNA ligase. The reaction mixture was used for transforming Escherichia coli 294 and, from an ampicillin-resistant transformant, a plasmid, pHBV P31 DNA, composed of the above three DNAs ligated together was obtained (cf. Fig. 9).

50 µg of said plasmid pHBVP31 was treated with 20 units each of the restriction enzymes SalI and ClaI in 100 µl of a reaction mixture [10 mM Tris-HCl (pH 7.5), 7 mM MgCl$_2$, 175 mM NaCl, 0.2 mM EDTA, 7 mM 2-mercaptoethanol] at 37°C for 3 hours, and the reaction mixture was subjected to electrophoresis using a 1.0% agarose slab gel, which was conducted under the conditions described in Reference Example 1. Thereafter, a 0.98 kb DNA fragment was separated from the gel by the method described in Reference Example 1 (cf. Fig. 9).

2 µg of said 0.98 kb DNA fragment was treated with DNA polymerase I large fragment under the conditions described in Reference Example 1 to render the cohesive ends blunt, followed by deproteinization with phenol and precipitation of a DNA with cold ethanol. To said 0.98 kb DNA fragment was joined a SalI linker [5'-P-d(CGTCGACG), Bethesda Research], followed by SalI treatment to render the cohesive ends blunt. Using a Sepharose 4B column, a fraction containing a 0.99 kb DNA fragment (subtype adw P31-encoding DNA) was collected and said DNA was precipitated with cold ethanol (cf. Fig. 9).

200 ng of the SalI-digested pPHO17-1 described in Example 3-(1) and 200 ng of the above 0.99 kb DNA fragment were ligated together under the conditions described in Reference Example 1 using T4 DNA ligase. Escherichia coli 294 was transformed using the reaction mixture, the transformants obtained were screened by the method described in Reference Example 1, and a strain (Escherichia coli 294/pPHO P31-W) carrying a plasmid, pPHO P31-W, having the 0.99 kb DNA fragment containing a subtype adw P31-encoding DNA as inserted therein in the same directionality as the PHO 5 promoter was isolated. Said plasmid was separated from said transformant by the alkaline extraction method and used for transforming the host yeast AH22R⁻ by the method of Hinnen et al. mentioned above, and a yeast transformant (Saccharomyces cerevisiae AH22R⁻/pPHO P31-W) carrying said plasmid was isolated (cf. Fig. 9).

A plasmid containing a subtype adw P31-encoding DNA and the PGK promoter or GLD promoter can also be constructed by the above procedure.

Example 5

Construction of recombinant DNA for expression in yeast of subtype adw hepatitis B virus surface antigen P31 gene and transformation of yeast with said DNA

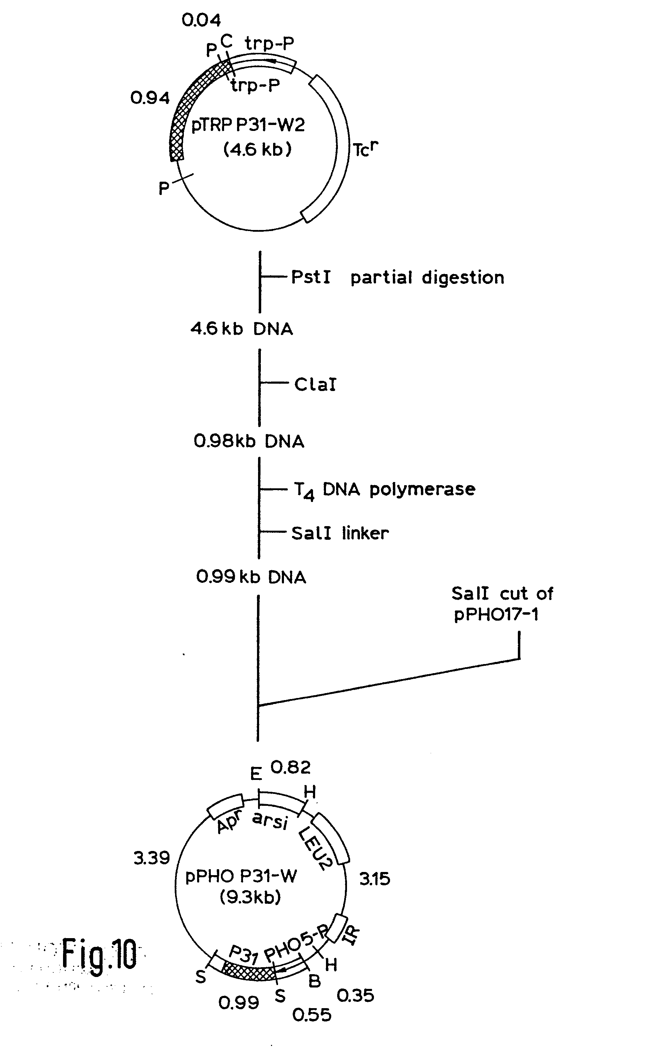

50 μg of the above-mentioned plasmid pTRP P31-W2 is treated with 20 units of the restriction enzyme PstI in 100 μl of a reaction mixture [10 mM Tris-HCl (pH 7.5), 10 mM MgCl$_2$, 50 mM NaCl, 1 mM dithiothreitol] at 37°C for 20 minutes for partial digestion, and the reaction mixture is subjected to electrophoresis using a 0.8% agarose slab gel under the conditions described in Reference Example 1. Thereafter, a 4.6 kb linear DNA molecule which is a product of cleavage with PstI only at one site is separated from the gel by the method described in Reference Example 1 (cf. Fig. 10).

5 µg of said 4.6 kb DNA molecule is treated with 5 units of the restriction enzyme ClaI in 30 µl of a reaction mixture [10 mM Tris-HCl (pH 7.5), 7 mM MgCl$_2$, 10 mM NaCl] at 37°C for 3 hours, and the reaction mixture is then subjected to 1.0% agarose slab gel electrophoresis under the conditions described in Reference Example 1. Thereafter, a 0.98 kb DNA fragment is separated from the gel by the method described in Reference Example 1.

0.8 µg of said 0.98 kb DNA fragment is treated in 20 µl of a reaction mixture [33 mM Tris-acetate (pH 7.9), 66 mM potassium acetate, 10 mM magnesium acetate, 5 mM dithiothreitol] using 2.5 units of T4 DNA polymerase at 37°C for 15 minutes to render the cohesive ends blunt, followed by deproteinization with phenol and precipitation of a DNA with cold ethanol. Using the method described in Example 3-(1), a SalI linker is joined to said 0.98 kb DNA fragment, followed by SalI treatment to render the cohesive end blunt. Using a Sepharose 4B column, a fraction containing a 0.99 kb DNA fragment (subtype adw P31-encoding DNA) is collected and said DNA is precipitated with cold ethanol.

200 ng of the SalI-digested pPHO17-1 described in Example 3-(1) and 200 ng of the above 0.99 kb DNA fragment are ligated together under the conditions described in Reference Example 1 using T4 DNA ligase. Using the reaction mixture, Escherichia coli 294 is transformed and the transformants obtained are screened by the method described in Reference Example 1, and a strain (294/pPHO P31-W) carrying a plasmid, pPHO P31-W) having the 0.99 kb DNA fragment containing a subtype adw P31-encoding DNA as inserted therein in the same directionality as the PHO 5 promoter is isolated. Said plasmid pPHO P31-W is separated from said transformant by the alkaline extraction method and used for transforming the host yeast AH22R⁻ by the method of Hinnen et al. mentioned above, and a yeast transformant (AH22R⁻/pPHOP31-W) carrying said plasmid is isolated (cf. Fig. 10).

When this transformant is cultivated by a conventional method, the desired P31 can be obtained.

Example 6 (1)

Expression of P31 gene in _Escherichia_ _coli_

Each transformant carrying a P31 gene expression plasmid as obtained in Example 1 or 2 was cultivated in M-9 medium containing 1.0% glucose and 1.0% casamino acids at 37°C for 6 hours and, then, cells were harvested and washed with a buffer [30 mM Tris-HCl (pH 8.0), 50 mM NaCl, 5 mM EDTA]. The cells were suspended in a lyzing solution comprising 10 mM Tris-HCl (pH 8.0), 5 mM EDTA, 1 mM phenylmethylsulfonyl fluoride and 5 mg/ml lysozyme and lysis was effected. To said lysate was added guanidine hydrochloride to a final concentration of 5 M. After incubation at 37°C for 2 hours, the lysate was centrifuged at 15,000 rpm and room temperature for 15 minutes to give a supernatant. The P31 activity of this supernatant was determined by the direct immunoassay mentioned hereinabove. The results thus obtained
are shown in Table 2. The yields of P31 were each obtained
in terms of the yield per milliliter of broth. The
yields so calculated were found to be higher than the
surface antigen yields described in Japanese published
unexamined patent application No. 201796/1983.

Table 2

| Transformant | HBsAg (units/ml broth) |
|---|---|
| Escherichia coli 294/pTRP P31-R | 220 |
| Escherichia coli 294/pTRP P31-W2 | 300 |

One unit of HBsAg corresponds to the count value
obtained with that amount of the $^{125}$I-anti-HBsAg anti-
body attached to the Ausria II-125 kit which is bound
to 1 ng of HBsAg small particles.

Example 6 (2)

Expression of P31 gene in yeast

Each yeast transformant obtained in Example 3 or
4 and carrying a P31 gene expression plasmid was
cultivated in Burkholder medium or a low phosphate level
modification thereof at 30°C for 2 days and, thereafter,
cells were harvested and washed with physiological saline.

The cells were converted to the spheroplast state
by treatment with Zymolyase [Seikagaku Kogyo] according
to the method of Miyanohara, A. et al. Thereto was
added 0.1% Triton X-100 for extraction of P31. The
lysate was centrifuged at room temperature at 15,000 rpm for 15 minutes to give a supernatant. This supernatant was assyed for P31 activity using Auszyme II [Abbott]. The results obtained are shown in Table 3. The yields of P31 were each calculated in terms of the yield per liter of broth.

Table 3

|  | P31 (µg/ broth) |
| --- | --- |
| Saccharomyces cerevisiae AH22R⁻/pPHO P31-R | 1200 |
| Saccharomyces cerevisiae AH22R⁻/pPHO P31-W | 1100 |

P31 was assayed using Auszyme II with HBsAg particles as the standard.

Example 7

Production of substantially pure P31 protein (1) Extraction from cells

100 g of Escherichia coli 294/pTRP P31-R cells obtained by cultivating by the method described in Example 6 and freezing at -20°C and stored in the frozen state were uniformly suspended in 200 ml of a buffer (pH 7.5) containing 10 mM EDTA and 25 mM sodium phosphate. To this suspension were added 696 mg of phenylmethylsulfonyl fluoride and 100 mg of lysozyme, and the mixture was warmed at 37°C for 15 minutes. To the mixture was added 200 ml of 25 mM sodium phosphate buffer (pH 7.5) containing 8 M urea, and the whole mixture was homogenized by two 30-second treatments at 4,000 rpm using an Omni-mixer (Servall). After further addition of 600 ml of 25 mM sodium phosphate buffer containing 8 M urea to the homegenate, the whole mixture was stirred at 4°C for 4 hours. To this lysate was further added 3,000 ml of 25 mM sodium phosphate buffer (pH 7.5) and the resultant mixture was stirred for further 1 hour and then centrifuged at 18,900 x g for 70 minutes to give 3,900 ml of a supernatant ($9.7 \times 10^3$ units/ml).

(2)  Purification with poly-HSA-Cellulofine column

The supernatant obtained above was passed through the poly-HSA-Cellulofine column (5 x 2 cm), which was obtained in Reference Example 4 and equilibrated with 25 mM sodium phosphate buffer (pH 7.5),for adsorpion of the P31 protein thereon. The column was washed in sequence with 300 ml of PBS containing 0.05% Tween 20, 220 ml of PBS containing 0.05% Tween 20 and 0.5 M NaCl, 80 ml of PBS containing 0.05% Tween 20 and 300 ml of 25 mM sodium phosphate buffer (pH 7.5) containing 4 M urea and then P31 was eluted with 300 ml of a sodium phosphate buffer (pH 7.5) containing 7.5 M urea. The active fraction (250 ml) was concentrated to 7.2 ml ($2.3 \times 10^6$ units/ml) using a YM-5 memberane (Amicon, USA). To 7 ml of the obtained concentrate was added 140 mg of DTT and, after reduction at 80°C for 15 minutes, the concentrate was filtered through a membrane filter (ACRODISC; 0.2 μm in pore size; Gelman).

(3)  Purification by high-performance liquid chromatography using reversed-phase column

A 500-μl portion of the filtrate obtained above

was allowed to be adsorbed on an AP202 300 $\overset{\circ}{A}$ ($C_8$) reversed-phased column (YMC-Shimakyu) and high-performance liquid chromatography was performed using a trifluoroacetic acid-acetonitrile-n-propyl alcohol system as the eluent system.

Column, AP202 300 $\overset{\circ}{A}$ ($C_8$) (4.6 x 150 mm); column temperature, 30°C; eluent A, 0.1% trifuloroacetic acid-99.9% water; eluent B, 0.1% trifluoroacetic acid-49.95% acetonitrile-49.95% n-propyl alcohol; elution program, minute 0 (45% A + 55% B)-minute 25 (25% A + 75% B)-minute 47 (25% A + 75% B)-minute 49 (5% A + 95% B)-minute 50 (45% A + 55% B); elution rate, 1 ml/min.; detection wavelength, 280 nm. A fraction showing a retention time of about 41 minutes under these conditions (4.8 ml; 3.1 x $10^4$ units/ml) was collected and lyophilized. Thus was obtained a white powder containing 149 μg (1 x $10^6$ units/mg) of sunstantially pure P31 protein (subtype adr).

The substantially pure P31 protein (subtype adr) produced by the above method had the following characteristic properties:

(1) Homogeneity:

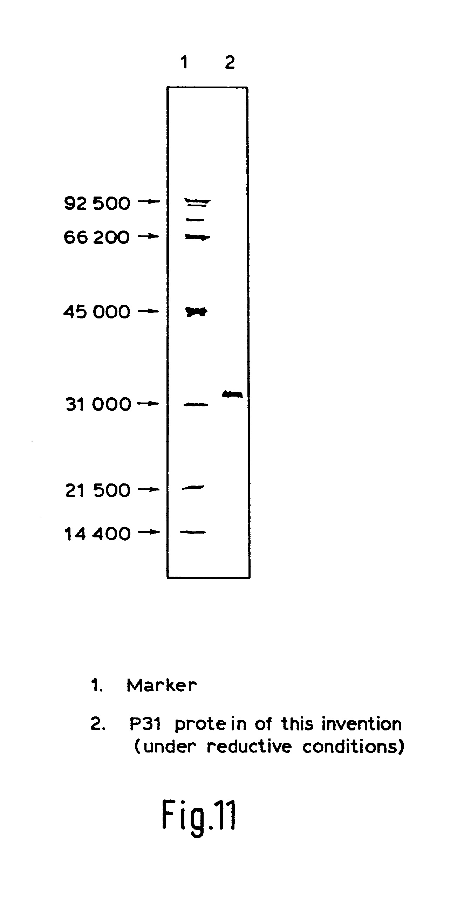

SDS-polyacrylamide slab gel electrophoresis was performed by the method of Laemmli [Nature, 227, 680 (1970)], followed by staining with the silver staining reagent "Daiichi" (Daiichi Kagaku, Tokyo) and the P31 protein was detected as a single band (cf. Fig. 11).

0171908

(2)  Molecular weight:

The molecular weight of the p31 protein was calculated to be about 32,000 daltons based on the result
of SDS-polyacrylamide slab gel electrophoresis (cf.
Fig. 11).

(3)  Amino acid composition:

In a glass tube for hydrolysis, there was
placed 20 µg of the P31 protein.  After addition of
constant boiling point hydrochloric acid containing
4% thioglycolic acid, the tube was sealed under
redcued pressure, and hydrolysis was performed at 110°C
for 24, 48, 72 or 96 hours.  After hydrolysis, the tube
was opened, the hydrochloric acid was removed under
reduced pressure, the residue was dissolved in 0.02 N
hydrochloric acid, and amino acid analysis was performed
using a Hitachi model 835 amino acid analyzer.  Cystine and cysteine
were determined, according to Hirs' method [Methods in Enzymol., 11,
197(1967)], as cysteric acid on the amino acid analyzer after performic
acid oxidation of the P31 protein and hydrolysis in constant boiling point
hydrochloric acid under reduced pressure for 24 hours.
The amino acid analysis data were calculated each as
an average of the values obtained by hydrolysis for 24,
48 and 72 hours.  For isoleucine, leucine and phenylalanine, however, the values obtained after 96 hours of
hydrolysis were employed and, for serine and threonine,
the values were determined by extrapolation to hour 0
(zero) of hydrolysis.  The results thus obtained are
shown in Table 4.

Table 4

| Amino acid | Molecule % |
|---|---|
| Asp/Asn | 5.8 |
| Thr | 7.8 |
| Ser | 9.6 |
| Glu/Gln | 5.3 |
| Pro | 11.5 |
| Gly | 7.0 |
| Ala | 4.7 |
| 1/2Cys | 3.9 |
| Val | 5.2 |
| Met | 2.5 |
| Ile | 5.2 |
| Leu | 12.7 |
| Tyr | 2.4 |
| Phe | 6.4 |
| Lys | 1.5 |
| His | 1.0 |
| Arg | 3.8 |
| Trp | 3.6 |

(4)   N-Terminal amino acid sequence:

The N-terminal amino acid sequence was analyzed by applying, to 62 µg of the P31 protein, the automatic Edman degradation method using a Gas-phase Protein Sequenator (Applied Biosystems model 470A, USA).  Phenyl-thiohydantoin-amino acids (PTH-amino acids) were identified by high-performance liquid chromatography using a Micro Pak C18-3 SP-DS column (Varian, USA).  The PTH-amino acids detected in the respective steps are shown in Table 5.

Table 5

| Step | PTH-amino acid detected |
|------|-------------------------|
| 1 | Methionine |
| 2 | Glutamine |
| 3 | Tryptophan |
| 4 | Asparagine |
| 5 | $X^{1)}$ |
| 6 | Threonine |

[1]  Unidentifiable.

(5)   C-Terminal amino acid:

520 µg of said P31 protein was placed in a glass tube for degradation with hydrazine  and 0.1 ml of anhydrous hydrazine was added. The tube was sealed under reduced pressure, and the whole was heated at 100°C for 6 hours.  The degradation product obtained was lyophilized and dissolved in distilled water.  Benzaldehyde was added to that solution and the resulting mixture was stirred at room temperature for 1 hour and, then, centrifuged to give a supernatant. This supernatant was lyophilized and subjected to amino acid analysis using a Hitachi model 835 amino acid analyzer.  As a result, 47 nanomoles of isoleucine was detected.

Also the use of cells of <u>Escherichia</u> <u>coli</u> 294/pTRP P31-W2 as obtained by cultivating said strain by the method described in Example 6 can lead to production of the P31 protein (subtype adw) in substantially pure form in the same manner as above.

Example 8

1 µg of the expression vector pPKT700-1 DNA described in Reference Example 2-(3) was treated with 2 units of the restriction enzyme SalI at 37°C for 2 hours and then further treated with 0.1 unit of alkaline phosphatase under the conditions described in Example 3-(1). Thereafter, a DNA was precipitated with cold ethanol (SalI-digested pPKT700-1).

Then, 200 ng of the 1.43 kb DNA fragment described in Example 3-(1) and 200 ng of the SalI-digested pPKT700-1 were ligated together under the conditions described in Reference Example 1 using T4 DNA ligase. Using the reaction mixture and following the procedure described in Example 3-(1), a strain (294/pPKT P31-R) carrying a plasmid, pPKT P31-R, with the 1.43 kb DNA fragment containing a subtype adr P31-encoding DNA inserted therein in the same directionality as the PGK promoter was isolated. The host yeast strain AH22R⁻ was transformed with said plasmid, and a yeast transformant (AH22R⁻/pPKT P31-R) was isolated (cf. Fig. 8).

Example 9

1 µg of the expression vector pGLD906-1 DNA described in Reference Example 3-(1) was treated with 2 units of the restriction enzyme SalI at 37°C for 2 hours and then further treated with 0.1 unit of alkaline phosphatase under the conditions described in Example 3-(1). Thereafter, a DNA was precipitated with cold ethanol SalI-digested pGLD906-1).

Then, 200 ng of the 1.43 kb DNA fragment described in Example 3-(1) and the SalI-digested pGLD906-1 were ligated together under the conditions described in Reference Example 1 using T4 DNA ligase. By the method describe in Example 3-(1) using the reaction mixture, a strain (294/pGLD P31-R) carrying a plasmid, pGLD P31-R, with the 1.43 kb DNA fragment containing a subtype adr P31-encoding DNA inserted therein in the same directionality as the GLD promoter was isolated. The host yeast AH22R⁻ was transformed with said plasmid, and a yeast transformant (AH22R⁻/pGLD P31-R) is obtained (cf. Fig. 8).

Example 10

Expression of P31 gene in yeast

Each yeast transformant obtained in Example 8 or 9 and carrying a P31 gene expression plasmid was cultivated in Burkholder medium or a low phosphate level modification thereof at 30°C for 2 days and, thereafter, cells were harvested and washed with physiological saline.

The cells were converted to the spheroplast state by treatment with Zymolyase [Seikagaku Kogyp] according to the method of Miyanohara, A. et al. Thereto was added 0.1% Triton X-100 for extraction of P31. The lysate was centrifuged at room temperature at 15,000 rpm for 15 minutes to give a supernatant. This supernatant was assayed for P31 activity using Auszyme II [Abbott]. The results obtained are shown in Table 6. The fields of P31 were each calculated in terms of the yield per liter of broth.

Table 6

|  | P31 (µg/$l$ broth) |
| --- | --- |
| Saccharomyces cerevisiae AH22R⁻/pGLD P31-R | 2400 |
| Saccharomyces cerevisiae AH22R⁻/pPKT P31-R | 350 |

P31 was assayed using Auszyme II with HBsAg particles as the standard.

Example 11

Plasmids were extracted from Escherichia coli 294 carrying the plasmid for expression of P31 gene as obtained in Examples 1 and 2, and Escherichia coli C600 were transformed with said plasmids to obtain Excherichia coli C600/pTRP P31-R and Escherichia coli C600/pTRP P31-W2.

Example 12

Escherichia coli C600 strain carrying a P31 gene expression plasmid as obtained in Example 11 was cultivated in M-9 medium containing 2.0% glucose and 1.0% casamino acids at 37°C for 8 hours and, then, cells were harvested and washed with a buffer [30 mM Tris-HCl (pH 8.0), 50 mM NaCl, 5 mM EDTA]. The cells were suspended in a lyzing solution comprising 10 mM Tris-HCl (pH 8.0), 5 mM EDTA, 1 mM phenylmethylsulfonyl fluoride and 5 mg/ml lysozyme and lysis was effected. To said lysate was added guanidine hydrochloride to a final concentration of 7 M. After incubation at 37°C for 2 hours, the lysate was centrifuged at 15,000 rpm and room temperature for 15 minutes to give a supernatant. The P31 activity of this supernatant was determined by the direct immunoassay mentioned hereinabove. The results thus obtained are shown in Table 7. The yields of P31 were each calculated in terms of the yield per milliliter of broth.

Table 7

| Transformant | HBsAg (units/ml broth) |
|---|---|
| Escherichia coli C600/pTRP P31-R | 1500 |
| Escherichia coli C600/pTRP P31-W2 | 1300 |

One unit of HBsAg corresponds to the count value obtained with that amount of the $^{125}$I-anti-HBsAg antibody attached to the Ausria II-125 kit which is bound to 1 ng of HBsAg small particles.

Example 13

Production of substantially pure P31 protein

The concentrate (0.6 ml, $2.1 \times 10^6$ units/ml) of the eluate from poly-HSA-Cellulofine column as obtained by the method described in Example 7 using Escherichia coli C600/ pTRP P31-R, was added 6 mg of DTT and, after reduction at 37°C for 1 hour, the mixture was filtered through a membrane filter. A 500-μl portion of the filtrate was allowed to be adsorbed on an AP224 300 Å ($C_8$) reversed-phased column (YMC-Shimakyu) and high-performance liquid chromatography was performed using a trifluoroacetic acid-acetonitrile-n-propyl alcohol system as the eluent system.

Column, AP224 300 $\overset{\circ}{A}$ ($C_8$) (1 x 30 cm); column temperature, 30°C; eluent A, 0.1% trifuloroacetic acid-99.9% water; eluent B, 0.1% trifuloroacetic acid-49.95% acetonitrile-49.95% n-propyl alcohol; elution program, minute 0 (70% A + 30% B)-minute 10 (28% A + 72% B)-minute 30 (24% A + 76% B)-minute 50 (14% A + 86% B)-minute 55 (5% A + 95% B)-minute 58 (5% A + 95% B)-minute 59 (70% A + 30% B); elution rate, 2.5 ml/min.; detection wavelength, 280 nm. A fraction showing a retention time of about 42 minutes under these conditions (20 ml; 1.7 x $10^4$ units/ml) was collected and reserved at -20°C.

The substantially pure P31 protein (subtype adr) produced by the above method had the same characteristic properties as P31 protein (subtype adr) obtained in Example 7.

Example 14

(1) 1 mg of P31 protein obtained in Example 13 was put into a glass tube, and the solvent was removed under reduced pressure. According to phenol-sulfuric acid method [Analytical Chemistry 28, 350 (1956)], quantitative analysis of carbohydrate was performed using glucose as a standard. The result was that carbohydrate detected in the sample was not greater than 0.3 molecule per molecule of P31 protein on calculating the molecular weight of P31 protein as 31,000 daltons Therefore, the obtained P31 protein seems to have no carbohyrate.

(2) In a glass tube for hydrolysis, there was placed 30 μg of the P31 protein obtained in Example 13, followed by removal of the solvent. After addition of trifluoroacetic

acid/constant boiling point hydrochloric acid [1:2 (v/v)],
the tube was sealed under reduced pressure, and hydrolysis
was performed at 170°C for 25, 50 and 180 minutes.  After
hydrolysis, the tube was opened, the trifluoroacetic acid
and hydrochloric acid were removed, the residue was dissolved
in 0.02 N hydrochloric acid, and amino acid analysis was
performed using a Hitachi model 835 amino acid analyzer.
Cystine and cysteine were determined, according to the method
of Hirs as cysteic acid on the amino acid analyzer after
performic acid oxidation of the P31 protein and hydrolysis
in constant boiling point hydrochloric acid under reduced
pressure for 24 hours.  Quantitative analysis for threonine,
serin and tryptophan was performed after hydrolysis of P31
protein in constant boilind hydrochloric acid containing 4%
thioglycolic  acid under reduced pressure for 16, 20, 24 and
30 hours.  The amino acid analysis data were calculated each
as an average of the values obtained by hydrolysis for 25, 50
and 180 minutes.  For tryptophan, however, the value
obtained after 24 hours of hydrolysis was employed and, for
serine and threonine, the values were determined by extrapolation to hour 0 (zero) of hydrolysis.  The results thus
obtained are shown in Table 8.

0171908

Table 8

| Amino acid | Molecule % |
|------------|------------|
| Asp/Asn | 5.5 |
| Thr | 7.9 |
| Ser | 10.3 |
| Glu/Gln | 4.7 |
| Pro | 11.9 |
| Gly | 7.3 |
| Ala | 4.4 |
| 1/2 Cys | 3.8 |
| Val | 4.7 |
| Met | 2.5 |
| Ile | 5.1 |
| Leu | 13.5 |
| Tyr | 2.3 |
| Phe | 6.7 |
| Lys | 1.2 |
| His | 0.9 |
| Arg | 3.4 |
| Trp | 3.7 |

Institutes at which microorganisms have been deposited and the accession numbers are shown in Table 9.

In the Table, "IFO" and "FRI" represent "Institute for Fermentation, Osaka" (Japan) and "Fermentation Research Institute, Agency of Industrial Science and Technology, Ministry of International Trade and Industry, Japan", respectively.  E. coli and S. cerevisiae represent Escherichia coli and Saccharomyces cerevisiae, respectively,

"FERM BP" number represents the accession number of a deposit under the Budapest Treaty and "FERM P" number in parentheses represents the accession number before conversion to a deposit under the Budapest Treaty.

0171908

Table 9

| Microorganism | IFO | FRI |
| --- | --- | --- |
| | IFO No. | FERM No. |
| E. coli 294 | 14171 | ————— |
| E. coli 294/pTRP P31-R | 14355 | BP-802 (P-7709) |
| E. coli 294/pTRP P31-W2 | 14356 | BP-803 (P-7710) |
| S. cerevisiae AH22R⁻ | 10134 | BP-804 (P-7824) |
| S. cerevisiae AH22R⁻/pPHO P31-R | 10135 | BP-805 (P-7825) |
| S. cerevisiae AH22R⁻/pPHO P31-W | 10136 | BP-806 (P-7826) |
| E. coli C600/pTRP P31-R | 14425 | BP-807 (P-8145) |
| S. cerevisiae AH22R⁻/pGLD P31-R | 10145 | BP-800 |
| S. cerevisiae AH22R⁻/pPKT P31-R | 10146 | BP-801 |
| E. coli C600 | 14410 | BP-808 (P-8154) |

E. coli 294 is a known strain [Backman, K. et al.,
Proc. Natl. Acad. Sci. USA, 73, 4174 (1974)].

HBsAg P31 obtained in accordance with this invention is
useful as a vaccine for diagnosis or prevention of HBV
infection.

What we claim is:

1.    A recombinant DNA wherein a DNA coding for hepatitis B virus surface antigen P31 is inserted into the 3' end of a promoter region.

2.    A recombinant DNA according to claim 1, in which the promoter is tryptophan promoter.

3.    A recombinant DNA according to claim 1, in which the promoter is repressible acid phosphatase promoter.

4.    A recombinant DNA according to claim 1, in which the promoter is glycelaldehyde 3-phosphate dehydrogenase promoter.

5.    A recombinant DNA according to claim 1, in which the promoter is 3-phosphoglycerate kinase promoter.

6.    A recombinant DNA according to claim 1, in which the hepatitis B virus surface antigen P31 is <u>adr</u> hepatitis B virus surface antigen P31.

7.    A recombinant DNA according to claim 1, in which the hepatitis B virus surface antigen P31 is <u>adw</u> hepatitis B virus surface antigen P31.

8.    A recombinant DNA according to claim 1, in which the hepatitis B virus surface antigen P31 in <u>adr</u> hepatitis B virus surface antigen P31 and the promoter is tryptophan promoter.

9.    A recombinant DNA according to claim 1, in which the hepatitis B virus surface antigen P31 is <u>adr</u> hepatitis B virus surface antigen P31 and the promoter is repressible acid phosphatase promoter.

10.    The recombinant DNA according to claim 1, which is pTRP P31-R.

11.    The recombinant DNA according to claim 1, which is pPHO P31-R.

12.    The recombinant DNA according to claim 1, which is pTRP P31-W2.

13.    The recombinant DNA according to claim 1, which is pPHO P31-W.

14.    The recombinant DNA according to claim 1, which is pGLD P31-R.

15.    The recombinant DNA according to claim 1, which is pPKT P31-R.

16.    A transformant which carries a recombinant DNA in which a DNA coding for hepatitis B virus surface antigen P31 is inserted into the 3' and of a promoter region.

17.    A transformant according to claim 16, which is a microorganism belonging to Escherichia coli.

18.    A transformant according to claim 16, which is a microorganism belonging to Saccharomyces cerevisiae.

19.    A transformant according to claim 16, which is a microorganism belonging to Escherichia coli 294.

20.    A transformant according to claim 16, which is a microorganism belonging to Escherichia coli C600.

21.   The transformant according to claim 16, which is
Escherichia coli 294/pTRP P31-R.

22.   The transformant according to claim 16, which is
Escherichia coli 294/pTRP P31-W2.

23.   The transformant according to claim 16, which is
Escherichia coli C600/pTRP P31-R.

24.   The transformant according to claim 16, which is
Saccharomyces cerevisiae AH22R⁻/pPHO P31-R.

25.   The transformant according to claim 16, which is
Saccharomyces cerevisiae AH22R⁻/pPHO P31-W.

26.   The transformant according to claim 16, which is
Saccharomyces cerevisiae AH22R⁻/pGLD P31-R.

27.   The transformant according to claim 16, which is
Saccharomyces cerevisiae AH22R⁻/pPKT P31-R.

28.   Non-glycosylated hepatitis B virus surface antigen P31
protein.

29.   Non-glycosylated adr hepatitis B virus surface antigen
P31 protein.

30.   A method for producing a recombinant DNA wherein a DNA
coding for hepatitis B virus surface antigen P31 is inserted
into the 3' end of a promoter region, which comprises
inserting the DNA coding for hepatitis B virus surface
antigen P31 into the 3' end of the promoter region.

31.   A method for producing a transformant which carries a
recombinant DNA wherein a DNA coding for hepatitis B virus
surface antigen P31 is inserted into the 3' end of a promoter
region, which comprises transforming a host with the recombinant
DNA as defined hereinbefore.

32.   A method for producing hepatitis B virus surface antigen
P31, which comprises (a) cultivating a transformant which
carries a recombinant DNA wherein a DNA coding for hepatitis
B virus surface antigen P31 is inserted into the 3' end of a
promoter region, (b) accemulating the hepatitis B virus surface
antigen P31 in the culture and (c) recovering the same.

33.   A method for producing a hepatitis B virus surface
antigen P31 protein, which comprises (a) cultivating a
transformant which carries a DNA having a base sequence coding
for hepatitis B virus surface antigen P31, (b) accumulating
the hepatitis B virus surface antigen P31 in the culture,
(c) collecting the P31-containing solution and (d) subjecting
the P31-containing solution to a purification process involving
affinity-chromatographic treatment.

34.   A method according to claim 33, wherein
the purification process involves afinity-chromatographic
treatment using polymerized human serum albumin as a ligand.

```
        1                          28
        CCTCTAAGAGACAGTCATCCTCAGGCCATG CAG TGG AAT TCC ACA ACA TTC CAC CAA GCT
  1                                  Met Gln Trp Asn Ser Thr Thr Phe His Gln Ala

        CTG CTA GAT CCC AGA GTG AGG GGC CTA TAT TTT CCT GCT GGT GGC TCC AGT TCC
 12     Leu Leu Asp Pro Arg Val Arg Gly Leu Tyr Phe Pro Ala Gly Gly Ser Ser Ser

        CGA ACA GTA AAC CCT GTT CCG ACT ACT GCC TCA CCC ATA TCG TCA ATC TTC TCG
 30     Gly Thr Val Asn Pro Val Pro Thr Thr Ala Ser Pro Ile Ser Ser Ile Phe Ser

        AGG ACT GGG GAC CCT GCA CCG AAC ATG GAG AAC ACA ACA TCA GGA TTC CTA GGA
 48     Arg Thr Gly Asp Pro Ala Pro Asn Met Glu Asn Thr Thr Ser Gly Phe Leu Gly

        CCC CTG CTC GTG TTA CAG GCG GGG TTT TTC TTG TTG ACA AGA ATC CTC ACA ATA
 66     Pro Leu Leu Val Leu Gln Ala Gly Phe Phe Leu Leu Thr Arg Ile Leu Thr Ile

        CCA CAG AGT CTA GAC TCG TGG TGG ACT TCT CTC AAT TTT CTA GGG GGA GCA CCC
 84     Pro Gln Ser Leu Asp Ser Trp Trp Thr Ser Leu Asn Phe Leu Gly Gly Ala Pro

        ACG TGT CCT GGC CAA AAT TCG CAG TCC CCA ACC TCC AAT CAC TCA CCA ACC TCT
102     Thr Cys Pro Gly Gln Asn Ser Gln Ser Pro Thr Ser Asn His Ser Pro Thr Ser

        TGT CCT CCA ATT TGT CCT GGC TAT CGC TGG ATG TGT CTG CGG CGT TTT ATC ATA
120     Cys Pro Pro Ile Cys Pro Gly Tyr Arg Trp Met Cys Leu Arg Arg Phe Ile Ile

        TTC CTC TTC ATC CTG CTG CTA TGC CTC ATC TTC TTG TTG GTT CTT CTG GAC TAC
138     Phe Leu Phe Ile Leu Leu Leu Cys Leu Ile Phe Leu Leu Val Leu Leu Asp Tyr

        CAA GGT ATG TTG CCC GTT TGT CCT CTA CTT CCA GGA ACA TCA ACC ACC AGC ACG
156     Gln Gly Met Leu Pro Val Cys Pro Leu Leu Pro Gly Thr Ser Thr Thr Ser Thr

        GGG CCA TGC AAG ACC TGC ACG ATT CCT GCT CAA GGA ACC TCT ATG TTT CCC TCT
174     Gly Pro Cys Lys Thr Cys Thr Ile Pro Ala Gln Gly Thr Ser Met Phe Pro Ser

        TGT TGC TGT ACA AAA CCT TCG GAC GGA AAC TGC ACT TGT ATT CCC ATC CCA TCA
192     Cys Cys Cys Thr Lys Pro Ser Asp Gly Asn Cys Thr Cys Ile Pro Ile Pro Ser

        TCC TGG GCT TTC GCA AGA TTC CTA TGG GAG TGG GCC TCA GTC CGT TTC TCC TGG
210     Ser Trp Ala Phe Ala Arg Phe Leu Trp Glu Trp Ala Ser Val Arg Phe Ser Trp

        CTC AGT TTA CTA GTG CCA TTT GTT CAG TGG TTC GTA GGG CTT TCC CCC ACT GTT
228     Leu Ser Leu Leu Val Pro Phe Val Gln Trp Phe Val Gly Leu Ser Pro Thr Val

        TGG CTT TCA GTT ATA TGG ATG ATG TGG TAT TGG GGG CCA AGT CTG TAC AAC ATC
246     Trp Leu Ser Val Ile Trp Met Met Trp Tyr Trp Gly Pro Ser Leu Tyr Asn Ile

        TTG AGT CCC TTT TTA CCT CTA TTA CCA ATT TTC TTT TGT CTT TGG GTA TAC ATT
264     Leu Ser Pro Phe Leu Pro Leu Leu Pro Ile Phe Phe Cys Leu Trp Val Tyr Ile

              873
        TAA ACCCT
282     ***
```

Fig.1

```
        1         10
        CCTCAGGGCATG CAG TGG AAT TCC ACT GCC TTG CAC CAA GCT CTG CAG GAT CCC AGA
1                 Met Gln Trp Asn Ser Thr Ala Leu His Gln Ala Leu Gln Asp Pro Arg

        GTC AGG GGT CTG TAT CTT CCT GCT GGT GGC TCC AGT TCA GGA ACA GTA AAC CCT
17      Val Arg Gly Leu Tyr Leu Pro Ala Gly Gly Ser Ser Ser Gly Thr Val Asn Pro

        GCT CCG AAT ATT GCC TCT CAC ATC TCG TCA ATC TCC GCG AGG ACT GGG GAC CCT
35      Ala Pro Asn Ile Ala Ser His Ile Ser Ser Ile Ser Ala Arg Thr Gly Asp Pro

        GTG ACG ATC ATG GAG AAC ATC ACA TCA GGA TTC CTA GGA CCC CTG CTC GTG TTA
53      Val Thr Ile Met Glu Asn Ile Thr Ser Gly Phe Leu Gly Pro Leu Leu Val Leu

        CAG GCG GGG TTT TTC TTG TTG ACA AGA ATC CTC ACA ATA CCG CAG AGT CTA GAC
71      Gln Ala Gly Phe Phe Leu Leu Thr Arg Ile Leu Thr Ile Pro Gln Ser Leu Asp

        TCG TGG TGG ACT TCT CTC AAT TTT CTA GGG GGA TCA CCC GTG TGT CTT GGC CAA
89      Ser Trp Trp Thr Ser Leu Asn Phe Leu Gly Gly Ser Pro Val Cys Leu Gly Gln

        AAT TCG CAG TCC CCA ACC TCC AAT CAC TCA CCA ACC TCC TGT CCT CCA ATT TGT
107     Asn Ser Gln Ser Pro Thr Ser Asn His Ser Pro Thr Ser Cys Pro Pro Ile Cys

        CCT GGT TAT CGC TGG ATG TGT CTG CGG CGT TTT ATC ATA TTC CTC TTC ATC CTG
125     Pro Gly Tyr Arg Trp Met Cys Leu Arg Arg Phe Ile Ile Phe Leu Phe Ile Leu

        CTG CTA TGC CTC ATC TTC TTA TTG GTT CTT CTG GAT TAT CAA GGT ATG TTG CCC
143     Leu Leu Cys Leu Ile Phe Leu Leu Val Leu Leu Asp Tyr Gln Gly Met Leu Pro

        GTT TGT CCT CTA ATT CCA GGA TCA ACA ACA ACC AGT ACG GGA CCA TGC AAA ACC
161     Val Cys Pro Leu Ile Pro Gly Ser Thr Thr Thr Ser Thr Gly Pro Cys Lys Thr

        TGC ACG ACT CCT GCT CAA GGC AAC TCT AAG TTT CCC TCA TGT TGC TGT ACA AAA
179     Cys Thr Thr Pro Ala Gln Gly Asn Ser Lys Phe Pro Ser Cys Cys Cys Thr Lys

        CCT ACG GAT GGA AAT TGC ACC TGT ATT CCC ATC CCA TCG TCC TGG GCT TTC GCA
197     Pro Thr Asp Gly Asn Cys Thr Cys Ile Pro Ile Pro Ser Ser Trp Ala Phe Ala

        AAA TAC CTA TGG GAG TGG GCC TCA GTC CGT TTC TCT TGG CTC AGT TTA CTA GTG
215     Lys Tyr Leu Trp Glu Trp Ala Ser Val Arg Phe Ser Trp Leu Ser Leu Leu Val

        CCA TTT GTT CAG TGG TTC GTA GGG CTT TCC CCC ACT GTT TGG CTT TCA GCT ATA
233     Pro Phe Val Gln Trp Phe Val Gly Leu Ser Pro Thr Val Trp Leu Ser Ala Ile

        TGG ATG ATG TGG TAT TGG GGG CCA AGT CTG TAC AGC ATC GTG AGT CCC TTT ATA
251     Trp Met Met Trp Tyr Trp Gly Pro Ser Leu Tyr Ser Ile Val Ser Pro Phe Ile

                                                           855
        CCG CTG TTA CCA ATT TTC TTT TGT CTC TGG GTA TAC ATT TAA ACCCT
269     Pro Leu Leu Pro Ile Phe Phe Cys Leu Trp Val Tyr Ile ***
```

Fig.2

Fig.3

Fig.4

4.3

Tc^r

Pr

pGLD 9
(6.5kb)

H

GLD

H

2.2

— Hind III

2.2kb DNA

H    HhaI  TaqI    GLD    H

0.75 | 0.36

0.69 | 0.5

HinfI   HinfI

— PolI
— BamHI linker
— HhaI

— HinfI

0.5kbDNA

— HhaI-TaqI

BamHI-HhaI 0.75kb DNA

0.36kbDNA

— PolI
— XhoI linker

BamHI-XhoI cut
of pSH19-1
(8.0kb)

HhaI-XhoI 0.36kb DNA

E 0.82

H

Ap^r  arsi

pGLD906
(9.1kb)

LEU2

3.71

3.15

GLD-P

IR

X

B   H

HhaI

0.36  0.75  0.35

— XhoI
— PalI
— SalI linker

E 0.82

H

Ap^r  arsi

pGLD906-1
(9.1kb)

LEU2

3.71

3.15

GLD-P

IR

S

B   H

HhaI

0.36  0.75  0.35

Fig.5

0171908

ClaI-BamHI cut
of pBR322
(4.01kb DNA)

EcoRI-BamHI
1.4kb DNA

P31 adaptor
5'CGATACAATGCAGTGG 3'
3'TATGTTACGTCACCTTAA 5'

BamHI
PolI
PstI linker

ClaI PstI cut
of pTRP 771
(3.3kb DNA

ClaI-PstI
1.42 kb DNA

Fig.6

Fig.7

0171908

Fig.8

Fig.9

Fig.10

1. Marker

2. P31 protein of this invention
   (under reductive conditions)

Fig.11